# EUROPEAN PATENT SPECIFICATION

(11) **EP 4 494 575 B1**
(45) Date of publication and mention of the grant of the patent: **11.11.2026**
(21) Application number: 23770623.9
(22) Date of filing: 09.03.2023
(51) Int. Cl.: A61B 17/135, A61B 17/132

(54) **HEMOSTASIS INSTRUMENT**
HÄMOSTASEINSTRUMENT
INSTRUMENT D'HÉMOSTASE

(30) Priority: 18.03.2022 JP 2022044656
(43) Date of publication of application: 22.01.2025
(73) Proprietor: Terumo Kabushiki Kaisha, Tokyo 151-0072 (JP)
(72) Inventor: KAWAMURA, Tomoya, Fujinomiya-shi, Shizuoka 418-0015 (JP); OUCHI, Tatsuya, Fujinomiya-shi, Shizuoka 418-0015 (JP); HIDAKA, Yuna, Fujinomiya-shi, Shizuoka 418-0015 (JP)
(74) Representative: Casalonga
(86) International application number: PCT/JP2023/008988
(87) International publication number: WO 2023/176667

(56) References cited:
- WO-A1-2020/111184
- WO-A1-2022/039272
- WO-A1-2022/210656

## Description

### Technical Field

The present invention relates to a hemostatic device.

### Background Art

As one of catheter procedures, a procedure is known in which various medical elongated bodies are introduced into a blood vessel of a limb such as an arm or hand of a patient through a puncture site formed by puncturing the blood vessel to perform diagnosis or therapy on a lesion site. For example, Patent Literature 1 discloses a hemostatic device for hemostasis at a puncture site formed to enable access to a blood vessel (including a distal radial artery) running in a hand.

The hemostatic device of Patent Literature 1 includes: a pressing member that applies a compressive force to a puncture site (hereinafter, also simply referred to as a "puncture site") formed on a hand of a patient, a plurality of band body portions for securing the pressing member to the hand of the patient, and a support member that presses the pressing member to the hand of the patient. In addition, the plurality of band body portions includes a first band body portion disposed at an inter-finger portion between fingers of the patient and a second band body portion and a third band body portion wrapped at a predetermined position on a forearm portion side of the hand of the patient.

### Citation List

### Patent Literature

Patent Literature 1: US 2019/0133602 A

WO 2022/039272 Al discloses a hemostatic instrument comprising a pressing member configured to compress a puncture site of a patient, a support member, and a plurality of band bodies. The support member includes a convex portion projecting away from one surface and an expansion member (such as a balloon) arranged on the opposite surface. The first and second band bodies are each connected to the pressing member via rotation axes, allowing them to rotate so as to approach or separate from the third band body. The support member further comprises a curved region that is convex in the direction of the convex portion. The expansion member is arranged to overlap the convex portion in the surface direction of the support member.

### Summary of Invention

### Technical Problem

An operator such as a medical doctor (hereinafter referred to as an "operator") may set the puncture site at a predetermined position avoiding a metacarpal bone of an index finger of the hand and a metacarpal bone of a thumb of the hand when forming the puncture site on the hand. By setting the puncture site at such a predetermined position, the operator can prevent a puncture device such as an indwelling needle from interfering with the metacarpal bone of each finger when forming the puncture site.

However, in a case where the puncture site is formed at the predetermined position avoiding the metacarpal bone of the index finger and the metacarpal bone of the thumb, when hemostasis is performed using the hemostatic device described in Patent Literature 1, the problems described below occur.

The operator disposes the pressing member and the support member of the hemostatic device of Patent Literature 1 so as to overlap the puncture site when hemostasis is performed on the puncture site. When the operator disposes the hemostatic device of Patent Literature 1 on the hand of the patient, the support member may be placed on the metacarpal bone of the index finger and the metacarpal bone of the thumb. In the hemostatic device of Patent Literature 1, when the second band body portion and the third band body portion are wrapped around the hand of the patient in a state where the support member is disposed on the hand of the patient as described above, a gap is formed between the pressing member and the hand, and the second band body portion and the third band body portion do not fit along an outer periphery of the hand of the patient. In a case where the hemostatic device is attached to the hand of the patient as described above, the pressing member and the support member can be firmly secured to the hand of the patient when the operator by strongly tightens the first band body portion to the inter-finger portion. On the other hand, when the operator tightens the first band body portion too strongly to the inter-finger portion, the patient may feel pain.
Therefore, in a case where the hemostatic device of Patent Literature 1 is used, when the first band body portion cannot be strongly tightened due to pain or the like of the patient, the support member is disposed in a state of being inclined in a direction of approaching a body surface of the hand from a fingertip side of the hand toward the forearm portion side.

In the case of hemostasis using the hemostatic device of Patent Literature 1, when the support member is disposed as described above, the pressing member cannot apply a compressive force to the puncture site in a vertical direction. Accordingly, the hemostatic device of Patent Literature 1 cannot effectively apply the compressive force to the puncture site.

In order to solve the problems described above, the inventors of the invention of the present application have studied to devise a shape of a support member included in a hemostatic device. As a result, the inventors have found that the support member can be disposed along the metacarpal bone of the index finger and the metacarpal bone of the thumb by configuring the support member such that the width of the support member decreases from an upper end portion of the support member disposed on the finger side toward a lower end portion of the support member disposed on the forearm portion side. Since the hemostatic device includes the support member formed in the shape as described above, the support member can be disposed on the hand in a stable state.

Further, the inventors have found that when the support member is configured such that the thickness decreases from the upper end portion of the support member toward the lower end portion of the support member, the support member can be disposed substantially parallel to the body surface of the hand by tightening the first band body portion extending from the upper end portion side of the support member to the inter-finger portion between the fingers with an appropriate tightening force. Since the hemostatic device includes the support member formed in the shape described above, the pressing member can apply a compressive force along a direction substantially perpendicular to the puncture site. Therefore, the hemostatic device enables the pressing member to effectively apply a compressive force to the puncture site.

Based on the above-described findings, the inventors have found that by further devising the shape of the support member, the pressing member can more effectively apply the compressive force to the puncture site while more effectively reducing a burden on the patient while the pressing member applies the compressive force to the puncture site.

An object of the present invention is to provide a hemostatic device capable of effectively applying a compressive force to a puncture site while reducing a burden on a patient even in the case of hemostasis at the puncture site formed at a predetermined position avoiding the metacarpal bone of the index finger of the hand and the metacarpal bone of the thumb of the hand.

### Solution to Problem

A hemostatic device according to the present invention includes: a cover member configured to cover a puncture site formed on a patient; and a pressing member connected to the cover member and configured to compress the puncture site, in which the cover member includes a main body portion where the pressing member is located, a first band body portion configured to be disposed between fingers of the patient and extending from the main body portion in a first direction, a second band body portion extending from the main body portion in a second direction different from the first direction, and a third band body portion facing the second band body portion across the pressing member and extending from the main body portion in a third direction different from the first direction and the second direction, the main body portion includes a support member formed of a material harder than the pressing member and configured to overlap the pressing member, the support member includes an upper end portion located on a first band body portion side and forming an end portion of the support member, a lower end portion forming an end portion opposite to the upper end portion, and a side surface portion connecting the upper end portion and the lower end portion, the support member is configured such that a width of the support member decreases from the upper end portion toward the lower end portion, and a thickness of the side surface portion decreases from the upper end portion toward the lower end portion, the lower end portion has a flat portion having a linear shape in plan view, and an outer surface of the support member is curved toward a second band body portion side and a third band body portion side.

### Advantageous Effects of Invention

The hemostatic device described above includes the support member made of a material harder than the pressing member. The support member is disposed so as to cover the puncture site together with the pressing member when hemostasis is performed on the puncture site formed at a predetermined position avoiding the metacarpal bone of the index finger of the hand of the patient and the metacarpal bone of the thumb of the hand of the patient. The support member can prevent the pressing member from lifting from the hand of the patient by pressing the pressing member to the hand of the patient. In addition, the width of the support member becomes shorter from the upper end portion toward the lower end portion. Therefore, when the support member is disposed on the hand of the patient, the support member can be disposed along the metacarpal bone of the index finger and the metacarpal bone of the thumb.

Therefore, in the hemostatic device described above, the second band body portion and the third band body portion can be firmly wrapped along an outer periphery of the hand of the patient. In addition, in the support member, the thickness of a side surface portion becomes thinner from the upper end portion toward the lower end portion. Additionally, the first band body portion configured to be disposed between fingers of the patient is configured to extend from the upper end portion side of the support member and press the upper end portion side of the support member to the hand of the patient. Therefore, the pressing member can appropriately apply a force to the upper end portion side of the support member when hemostasis starts in a state where the hemostatic device is attached to the hand of the patient. In addition, the support member lifts the upper end portion side of the support member using the force applied by the pressing member. Accordingly, the hemostatic device can dispose the support member such that an inner surface of the support member is substantially parallel to the body surface of the hand of the patient. Therefore, in the hemostatic device, the compressive force of the pressing member can be directed in a direction perpendicular to the puncture site by securing each band body portion to the hand of the patient in a state where the pressing member and the support member are disposed at the puncture site. Accordingly, the hemostatic device can effectively apply the compressive force to the puncture site.

Further, in the hemostatic device described above, the lower end portion of the support member has a flat portion having a linear shape in plan view. The flat portion having a linear shape located at the lower end portion of the support member prevents the lower end portion of the support member from biting into the body surface such as the wrist or the forearm portion of the patient when the patient performs a movement such as twisting the wrist toward the dorsal side of the hand in a state where the hemostatic device is attached to the hand of the patient. Therefore, the hemostatic device described above can suppress the patient from feeling pain even when the patient moves the wrist or the like while the pressing member applies the compressive force to the puncture site.

In addition, in the hemostatic device described above, the outer surface of the support member is curved toward the second band body portion side and the third band body portion side. Therefore, in the hemostatic device described above, when the operator wraps the second band body portion and the third band body portion connected to the main body portion of the cover member along the outer periphery of the hand of the patient, the second band body portion and the third band body portion are wrapped along the curved outer surface of the support member. Accordingly, the hemostatic device can increase the securing force of the support member and the pressing member to the hand of the patient.

### Brief Description of Drawings

Fig. 1 is a view illustrating a hemostatic device according to an embodiment, and is a plan view seen from an outer surface side of each band body portion.
Fig. 2 is a view illustrating the hemostatic device according to the embodiment, and is a plan view seen from an inner surface side of each band body portion.
Fig. 3 is an enlarged view illustrating a part of the hemostatic device as viewed from an outer surface side of a main body portion of a cover member.
Fig. 4 is an enlarged view illustrating a part of the hemostatic device as viewed from an inner surface side of the main body portion of the cover member.
Fig. 5 is an enlarged view illustrating a part of the hemostatic device as viewed from the outer surface side of the main body portion of the cover member.
Fig. 6 is a partial cross-sectional view of the hemostatic device taken along an arrow 6A-6A illustrated in Fig. 5, and is a view illustrating a state when an inflatable portion is inflated.
Fig. 7 is a partial cross-sectional view of the hemostatic device taken along an arrow 7A-7A illustrated in Fig. 5, and is a view illustrating a state when the inflatable portion is inflated.
Fig. 8 is a plan view of a support member.
Fig. 9 is a perspective view of the support member as viewed from an upper end portion side.
Fig. 10 is a perspective view of the support member as viewed from a lower end portion side.
Fig. 11 is a side view of the support member as viewed from a direction of an arrow 11A illustrated in Fig. 8.
Fig. 12 is a front view of the support member as viewed from a direction of an arrow 12A illustrated in Fig. 8.
Fig. 13 is a rear view of the support member as viewed from a direction of an arrow 13A illustrated in Fig. 8.
Fig. 14 is a perspective view of the support member.
Fig. 15 is a perspective view of the support member.
Fig. 16 is a view illustrating a hand (right hand) of a patient, which is a target of use of the hemostatic device.
Fig. 17 is a view schematically illustrating a usage example of the hemostatic device.
Fig. 18 is a view schematically illustrating a usage example of the hemostatic device.
Fig. 19 is a view schematically illustrating a usage example of the hemostatic device.
Fig. 20 is a partial cross-sectional view taken along an arrow 20A-20A illustrated in Fig. 19.
Fig. 21 is a partial cross-sectional view taken along an arrow 21A-21A illustrated in Fig. 19.
Fig. 22 is a view illustrating a right hand of a patient to which the hemostatic device is attached.
Fig. 23 is a partial cross-sectional view illustrating a part of the hemostatic device according to a modification.
Fig. 24 is a partial cross-sectional view illustrating a part of the hemostatic device according to a modification.

### Description of Embodiments

Hereinafter, an embodiment of the present invention will be described with reference to the accompanying drawings. The following description does not limit the technical scope or the significance of terms disclosed in the claims. In addition, dimensional ratios of the drawings are exaggerated for descriptive purposes and may differ from actual ratios. Herein, a range "X to Y" described in the present specification means "X or more and Y or less".

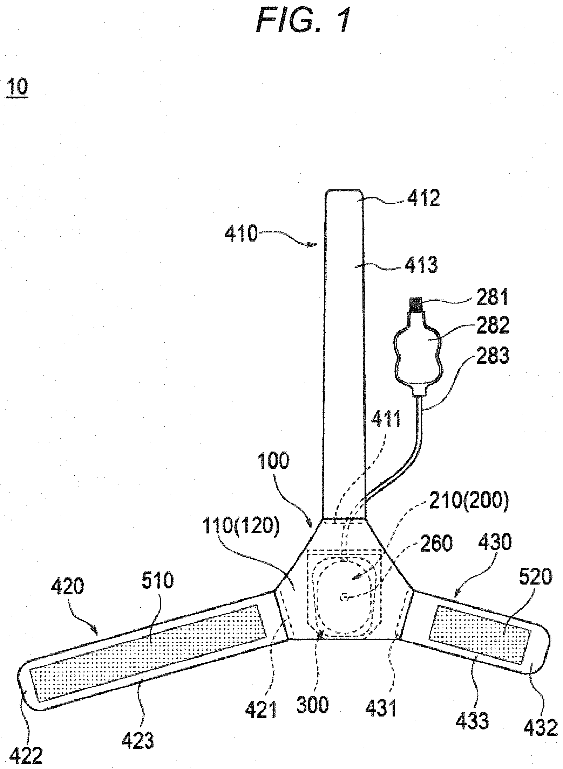

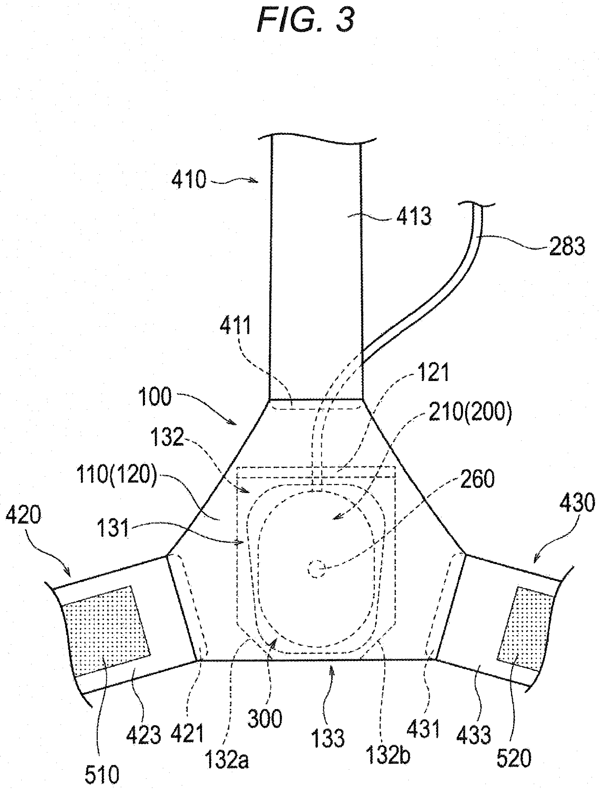

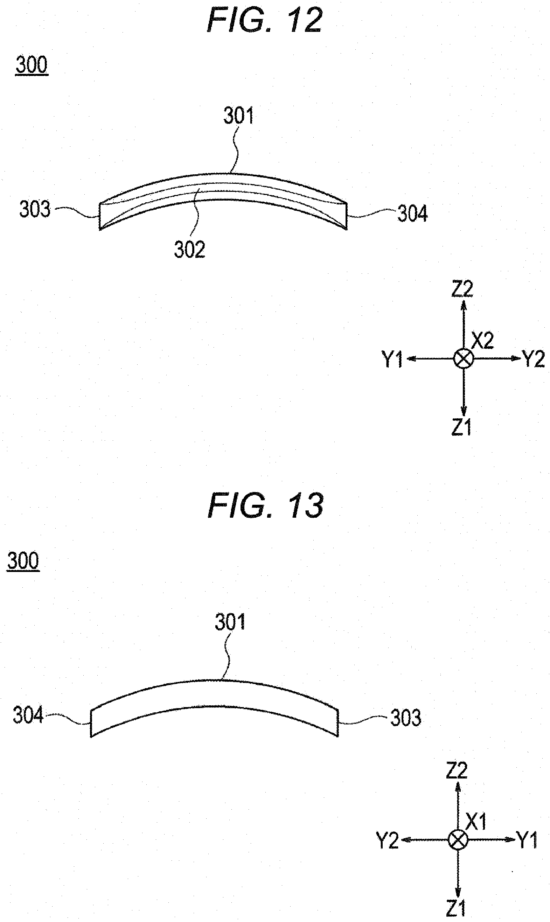

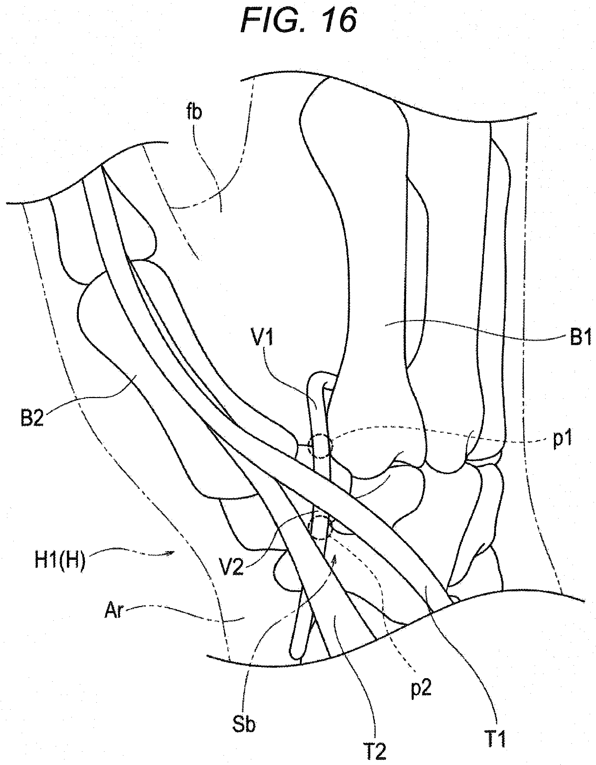

Figs. 1 to 15 are views for describing a hemostatic device 10 according to an embodiment. Figs. 16 to 22 are views for describing usage examples of the hemostatic device 10 according to the embodiment.

For example, as illustrated in Figs. 16, 19, 20, 21, and 22, the hemostatic device 10 can be used for hemostasis of a first puncture site p1 formed on a hand H located on a distal side (fingertip side) with respect to a forearm portion Ar of a patient when a sheath tube 610 of an introducer 600 indwelled in the first puncture site p1 is removed.

A specific position of the puncture site, which is a hemostasis target of the hemostatic device 10, is not particularly limited, but in the present embodiment, the first puncture site p1 and a second puncture site p2 are exemplified.

As illustrated in Figs. 16, 19, 20, 21, and 22, the first puncture site p1 is a puncture site formed in a distal radial artery (hereinafter, also referred to as a "blood vessel V1") located on a distal side of a snuff box Sb of a palmar artery running on a dorsal side of a right hand H1 located on the distal side with respect to the forearm portion Ar of the patient. In addition, the first puncture site p1 is located at a predetermined position, which is a position between a metacarpal bone B1 of an index finger and a metacarpal bone B2 of a thumb and avoiding the metacarpal bone B1 of the index finger and the metacarpal bone B2 of the thumb. Note that the snuff box Sb is a cavity in the hand located in the vicinity of the radius when the patient spreads the thumb of the hand H.

As illustrated in Fig. 16, the second puncture site p2 is a puncture site formed in an artery V2 located in the snuff box Sb of the palmar artery running on the dorsal side of the right hand H1. The second puncture site p2 is located on the forearm portion Ar side (proximal side) of the patient with respect to the first puncture site p1, and is located between an extensor pollicis longus muscle tendon T1 and an extensor pollicis brevis muscle tendon T2 located at the dorsal side of the right hand H1 of the patient. In addition, the second puncture site p2 is located at a predetermined position avoiding the position of the metacarpal bone B1 of the index finger and the position of the metacarpal bone B2 of the thumb.

Note that the hemostatic device 10 can also be applied to hemostasis of a puncture site on the left hand of the patient formed at a position corresponding to the first puncture site p1 exemplified in the right hand H1 of the patient or a puncture site on the left hand of the patient formed at a position corresponding to the second puncture site p2 exemplified in the right hand H1 of the patient.

Hereinafter, the hemostatic device 10 will be described in detail.

### <Hemostatic Device>

In brief, as illustrated in Figs. 1, 2, 19, 20, 21, and 22, the hemostatic device 10 includes a cover member 100 configured to cover the first puncture site p1 and a pressing member 200 connected to the cover member 100 and configured to compress the first puncture site p1.

### <Pressing Member>

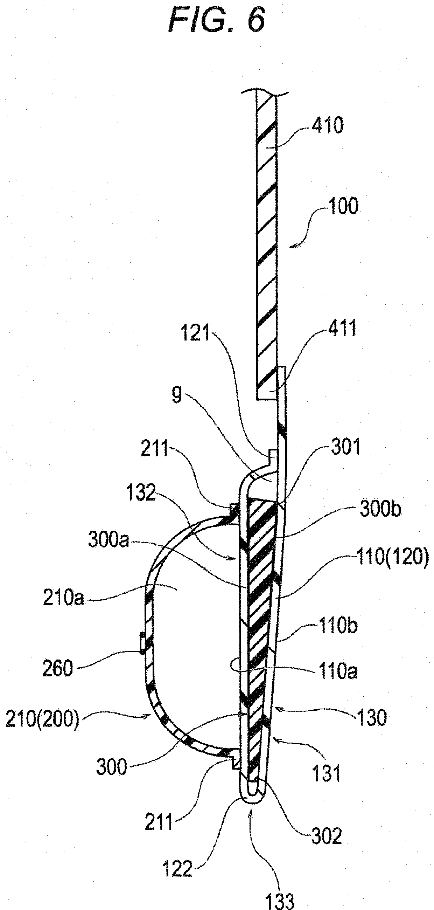

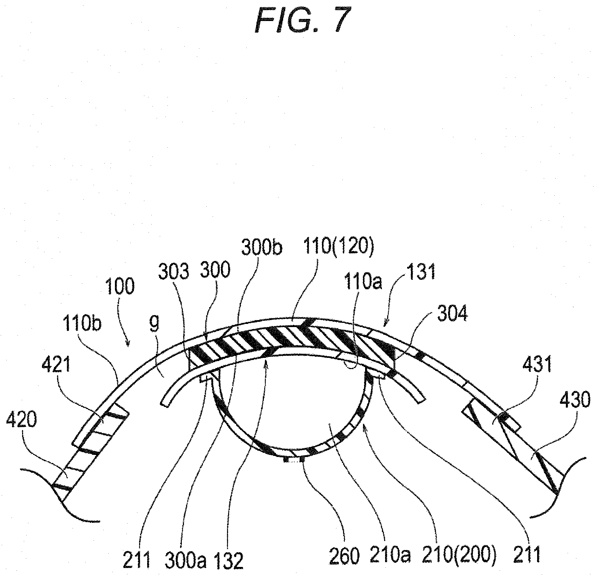

For example, as illustrated in Figs. 6 and 7, the pressing member 200 can include an inflatable member 210 that is inflatable by injection of a fluid. In the present embodiment, in the following description, an example in which the pressing member 200 includes the inflatable member 210 will be described.

The inflatable member 210 includes an inner cavity 210a into which a fluid can be injected. The inflatable member 210 is configured to inflate as a result of the injection of the fluid into the inner cavity 210a and deflate as a result of the discharge of the fluid injected into the inner cavity 210a. The fluid used to inflate the inflatable member 210 is, for example, a gas such as air. Figs. 6 and 7 illustrate cross-sectional views of the inflatable member 210 in an inflated state.

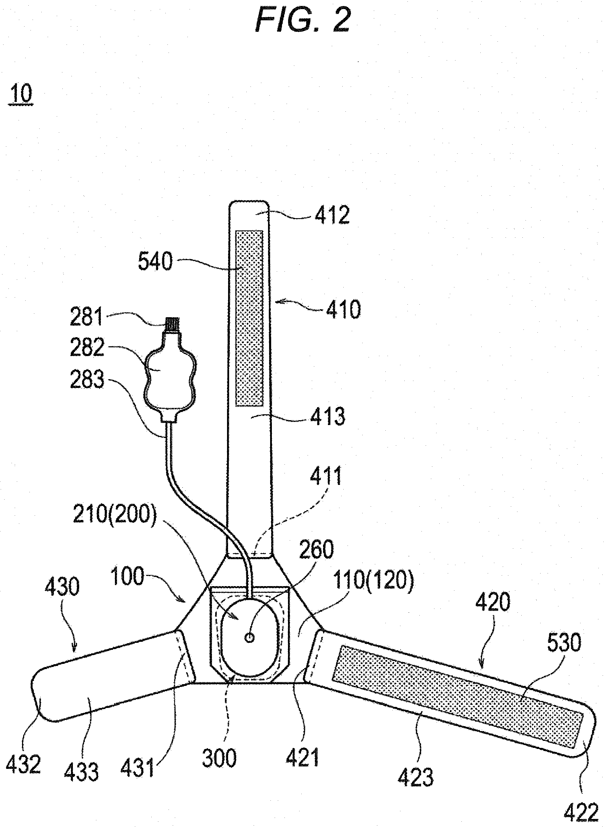

A tube 283 (see Figs. 1 and 2) to be described below is coupled to the inner cavity 210a of the inflatable member 210.

As illustrated in Figs. 6 and 7, the inflatable member 210 can include, for example, a film-like member (sheet material) connected to a sheet material 120 constituting a main body portion 110 of the cover member 100.

The inflatable member 210 may be disposed to form the inner cavity 210a with respect to a lower surface region 132 of a holding portion 130 formed by the sheet material 120.

An outer peripheral edge of the film-like member constitutes an edge portion 211 of the inflatable member 210. The edge portion 211 of the inflatable member 210 can be connected to the sheet material 120 by, for example, fusing, bonding, or the like.

The film-like member constituting the inflatable member 210 can be made of, for example, a resin material having a predetermined thickness.

A material for the film-like member constituting the inflatable member 210 is not particularly limited, and examples thereof include polyvinyl chloride, polyethylene, polypropylene, polybutadiene, polyolefin such as ethylenevinyl acetate copolymer (EVA), polyester such as polyethylene terephthalate (PET) or polybutylene terephthalate (PBT), various thermoplastic elastomers such as polyvinylidene chloride, silicone, polyurethane, polyamide elastomer, polyurethane elastomer or polyester elastomer, nylon, nylon elastomer, or any combination thereof (e.g., blended resin, polymer alloy, laminate, or the like).

Note that the specific configuration of the inflatable member 210 included in the hemostatic device 10 is not particularly limited. For example, the inflatable member 210 may be formed by joining an edge portion of one sheet-like member to another sheet-like member in a state where the inner cavity 210a is formed between the two sheet-like members. In such a case, the inflatable member 210 connects the surface of the one sheet-like member to the sheet material 120 constituting the main body portion 110 of the cover member 100. In addition, the inflatable member 210 can also include, for example, one bag-shaped member shaped to include the inner cavity 210a inside. In such a case, the inflatable member 210 connects a part of the surface of the bag-shaped member to the sheet material 120 constituting the main body portion 110 of the cover member 100.

As illustrated in Figs. 6 and 7, the inflatable member 210 is disposed on an inner surface 110a side of the main body portion 110 of the cover member 100.

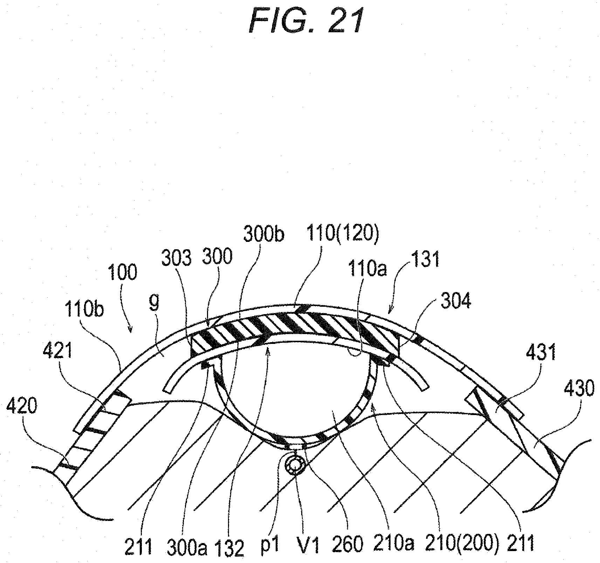

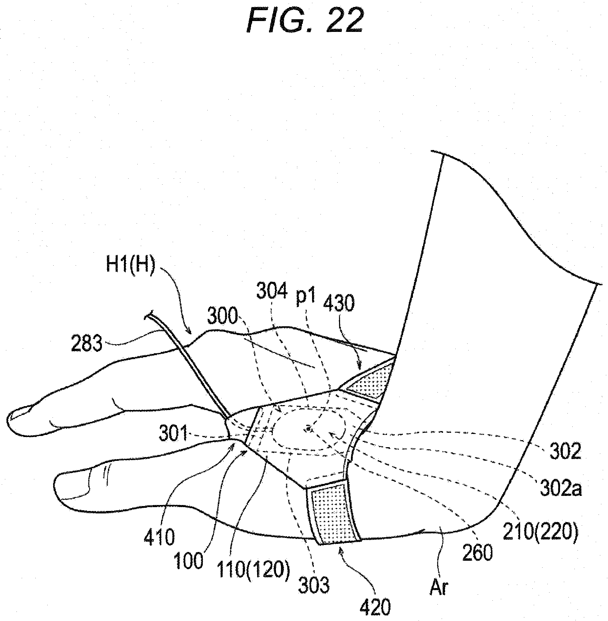

In the present embodiment, the inner surface 110a of the main body portion 110 includes a surface disposed on the body surface side of the hand H in the lower surface region 132 of the holding portion 130 when the hemostatic device 10 is attached on the hand H of the patient (see Figs. 21 and 22). An outer surface 110b of the main body portion 110 includes a surface on a side disposed facing the outside in an upper surface region 131 of the holding portion 130.

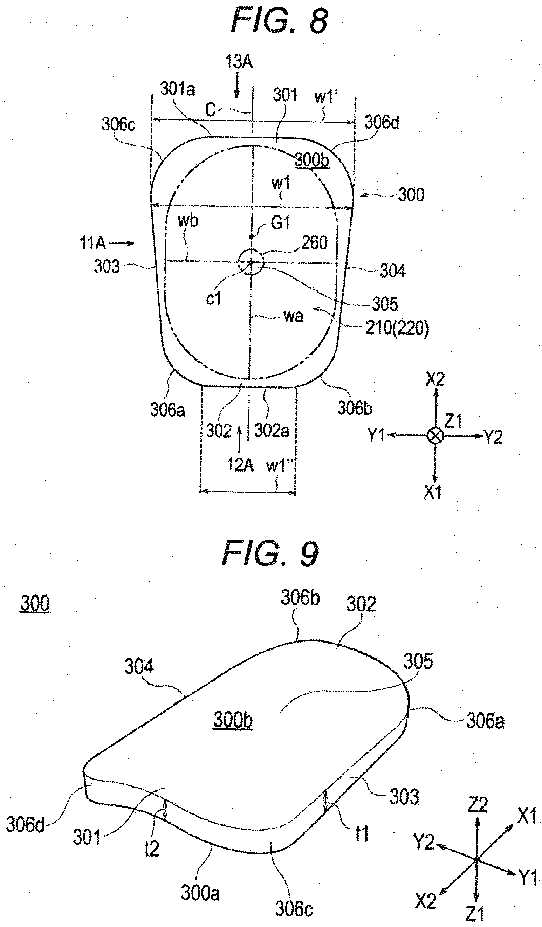

Fig. 8 illustrates a positional relationship between the inflatable member 210 in a deflated state and a support member 300 in plan view. The inflatable member 210 is indicated by the two-dot chain line.

The inflatable member 210 has a longitudinal width wa passing through a center c1 of the inflatable member 210 and a lateral width wb orthogonal to the longitudinal width wa and passing through the center c1 of the inflatable member 210 in a state where the inflatable member 210 is deflated. Note that the longitudinal width wa and the lateral width wb described above are dimensions that do not include the edge portion 211 located on the outer periphery of the inflatable member 210, and mean the longitudinal width wa and the lateral width wb in the outer shape of the inflatable member 210 illustrated in plan view illustrated in Fig. 8.

The center c1 of the inflatable member 210 is located at the center of the outer shape illustrated in plan view of Fig. 8. In the present embodiment, the center c1 is located at a position where a center position of an axis (long axis) along the longitudinal width wa and a center position of an axis (short axis) along the lateral width wb intersect. Note that, in the present embodiment, the center position of the axis (long axis) along the longitudinal width wa is a line segment passing through a point where distances from both ends of the axis along the longitudinal width wa are equal on the axis along the longitudinal width wa. In addition, the center position of the axis (short axis) along the lateral width wb is a line segment passing through a point where distances from both ends of the axis along the lateral width wb are equal on the axis along the lateral width wb.

The lateral width wb of the inflatable member 210 is shorter than the longitudinal width wa. In the present embodiment, the inflatable member 210 has a substantially oval shape that is rotationally symmetric relative to the center c1 in plan view illustrated in Fig. 8.

Note that the planar shape of the inflatable member 210 is not particularly limited. For example, the inflatable member 210 may be configured to have a planar shape such as a circular shape, an elliptical shape, or a rectangular shape. Even when the inflatable member 210 is configured to have a planar shape such as a circular shape, an elliptical shape, or a rectangular shape, the center of the inflatable member 210 can be defined by the center position of the outer shape in plan view.

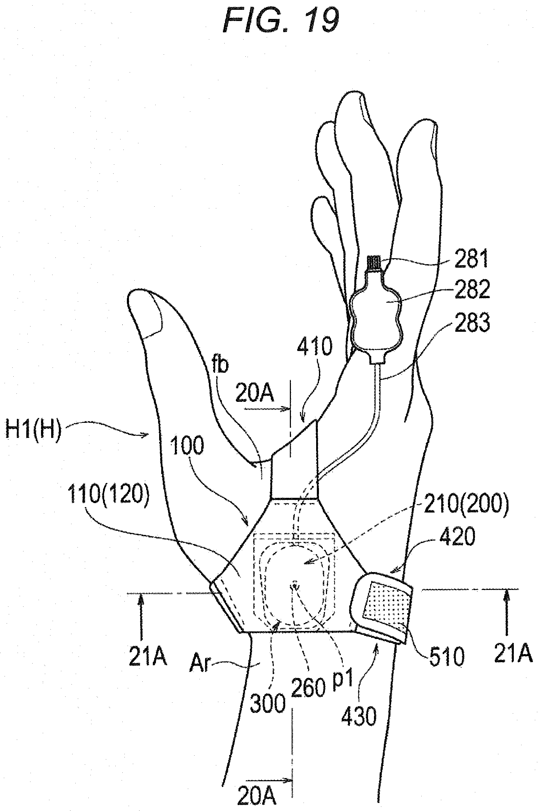

As illustrated in Figs. 19, 20, and 21, the inflatable member 210 includes a marker portion 260 for aligning the inflatable member 210 with the first puncture site p1.

As illustrated in Fig. 8, the marker portion 260 can include a transparent central portion surrounding the center c1 and a colored circular frame portion surrounding the central portion.

As illustrated in Figs. 6 and 7, the marker portion 260 is disposed on the outer surface of the inflatable member 210. Note that the marker portion 260 may be disposed on the inner surface facing the inner cavity 210a of the inflatable member 210.

In addition, the specific shape and color of the marker portion 260, the position of the inflatable member 210 in the plane direction, a forming method, and the like are not particularly limited. The marker portion 260 can also include, for example, a circular marker configured entirely to be colored, a marker including a transparent central portion and a rectangular frame portion, a rectangular marker configured entirely to be colored, or the like.

### <Cover Member>

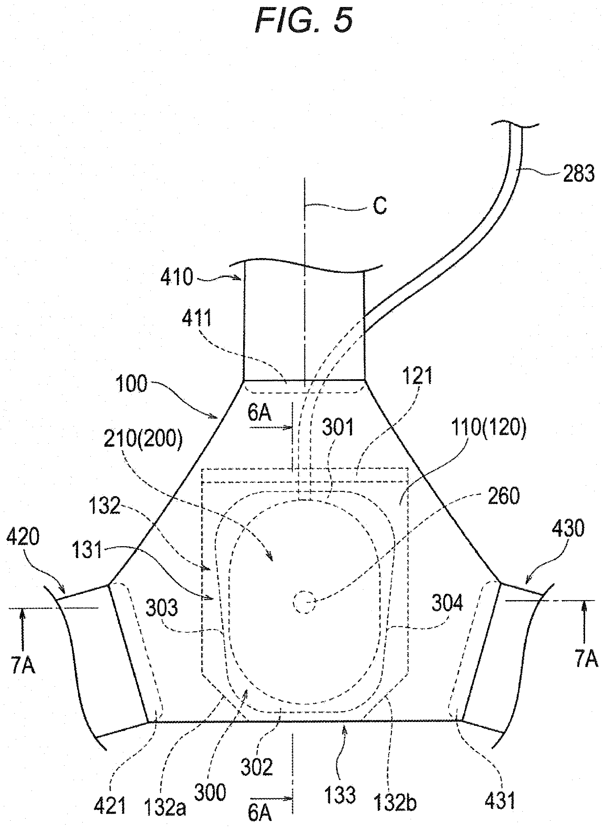

As illustrated in Figs. 5, 6, and 7, the cover member 100 includes the main body portion 110 where the inflatable member 210 is located, a first band body portion 410 configured to be disposed between fingers of the patient and extending from the main body portion 110 in a first direction, a second band body portion 420 extending from the main body portion 110 in a second direction different from the first direction, and a third band body portion 430 facing the second band body portion 420 across the inflatable member 210 and extending from the main body portion 110 in a third direction different from the first direction and the second direction.

A region of the cover member 100 where the inflatable member 210 is disposed (a region overlapping the inflatable member 210 in plan view illustrated in Fig. 5) constitutes the main body portion 110.

As illustrated in Figs. 5, 6, and 7, the main body portion 110 includes the support member 300 formed of a material harder than the inflatable member 210. The support member 300 is disposed so as to overlap the inflatable member 210 in plan view illustrated in Fig. 5. Details of the support member 300 will be described below.

As illustrated in Figs. 6 and 7, the main body portion 110 includes the holding portion 130 that holds the support member 300.

The holding portion 130 can include a part of the sheet material 120 constituting the cover member 100. In the present embodiment, a part of the sheet material 120 is folded back from the proximal side (the side disposed on the forearm portion Ar) to the distal side (the side disposed on the fingertip) such that a space portion g is formed at a position overlapping the main body portion 110 in plan view illustrated in Fig. 5. An end portion 121 located on the distal side of the folded-back portion of the sheet material 120 is connected to the main body portion 110.

As illustrated in Figs. 6 and 7, the holding portion 130 includes the upper surface region 131, the lower surface region 132 facing the upper surface region 131 across the space portion g, and a curved region 133 located at a proximal portion 122 of the main body portion 110.

The space portion g is closed between the end portion 121 and the proximal portion 122 in the longitudinal width wa direction (see the cross section illustrated in Fig. 6). In addition, in the space portion g, a second band body portion 420 side and a third band body portion 430 side communicate with the outside in the lateral width wb direction (see the cross section illustrated in Fig. 7).

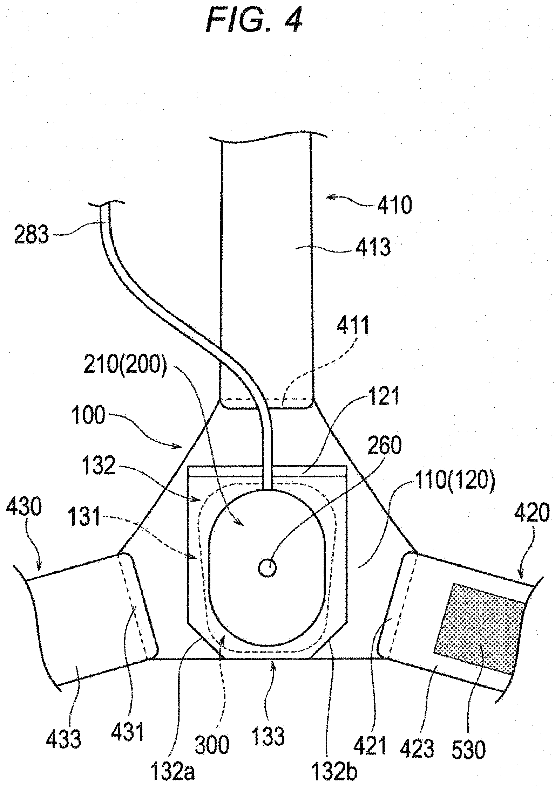

As illustrated in Figs. 4 and 5, constricted portions 132a and 132b in which the width of the lower surface region 132 gradually decreases toward the curved region 133 are formed in the vicinity of the curved region 133 of the holding portion 130 (in the vicinity of the proximal portion 122 of the main body portion 110).

As illustrated in Fig. 4, each of the band body portions 410, 420, and 430 can be connected to an arbitrary position in a region surrounding the periphery of the holding portion 130 in the main body portion 110. In addition, each of the band body portions 410, 420, and 430 can be connected to a surface disposed on the body surface side of the right hand H1 in the region described above. Each of the band body portions 410, 420, and 430 can be connected to the main body portion 110 by, for example, fusing or bonding. Note that each of the band body portions 410, 420, and 430 may be integrally configured with the main body portion 110.

As illustrated in Figs. 1 and 2, the first band body portion 410 includes one end portion 411 connected to the main body portion 110 of the cover member 100, an other end portion 412 located on the opposite side of the one end portion 411, and a main body portion 413 extending between the one end portion 411 and the other end portion 412.

For example, as illustrated in Fig. 19, the first band body portion 410 can be disposed so as to be hooked on an inter-finger portion fb located between the thumb and the index finger of the right hand H1 of the patient in a state where the inflatable member 210 is disposed at the first puncture site p1.

As illustrated in Figs. 1 and 2, the second band body portion 420 includes one end portion 421 connected to the main body portion 110 of the cover member 100, an other end portion 422 located on the opposite side of the one end portion 421, and a main body portion 423 extending between the one end portion 421 and the other end portion 422.

The second direction in which the second band body portion 420 extends is not particularly limited as long as it is a direction different from the first direction in which the first band body portion 410 extends.

As illustrated in Figs. 1 and 2, the third band body portion 430 includes one end portion 431 connected to the main body portion 110 of the cover member 100, an other end portion 432 located on the opposite side of the one end portion 431, and a main body portion 433 extending between the one end portion 431 and the other end portion 432.

The third direction in which the third band body portion 430 extends is not particularly limited as long as it is a direction different from the first direction in which the first band body portion 410 extends and the second direction in which the second band body portion 420 extends.

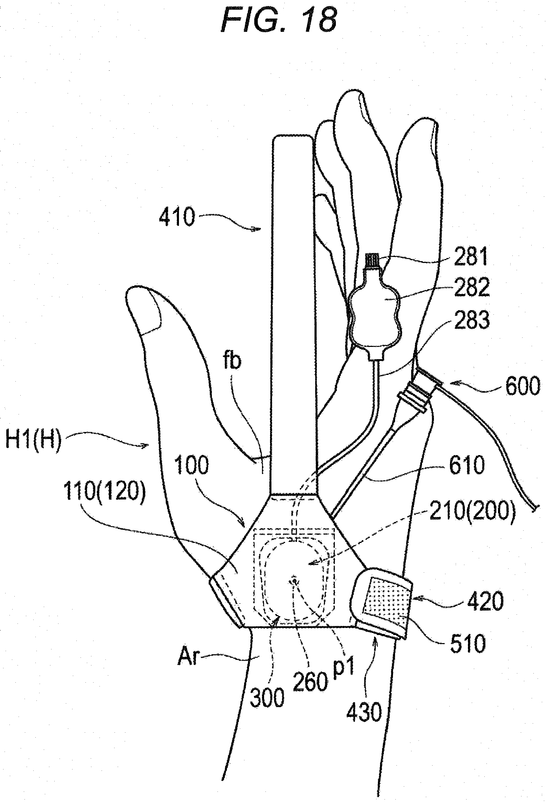

As illustrated in Figs. 18 and 19, the second band body portion 420 and the third band body portion 430 can be disposed so as to be wrapped along the outer periphery of the right hand H1 when the hemostatic device 10 is attached to the right hand H1 of the patient.

As illustrated in Fig. 5, the second band body portion 420 and the third band body portion 430 are located at positions facing each other across the inflatable member 210. Therefore, when the second band body portion 420 and the third band body portion 430 are wrapped around the right hand H1, a tensile force in a direction in which the second band body portion 420 and the third band body portion 430 move away from each other is applied to the second band body portion 420 and the third band body portion 430, and a tensile force in the same direction is also applied to the main body portion 110.

The constituent material of each of the band body portions 410, 420, and 430 is not particularly limited, and each of the band body portions 410, 420, and 430 can be made of, for example, a vinyl chloride resin, a polyurethane resin, a polyester resin, or the like. In addition, the shape, length, thickness, and the like of each of the band body portions 410, 420, and 430 are not particularly limited.

Four securing sites including a first securing site 510, a second securing site 520, a third securing site 530, and a fourth securing site 540 capable of securing the cover member 100 to the right hand H1 are disposed on each of the band body portions 410, 420, and 430.

As illustrated in Fig. 1, the first securing site 510 is disposed on the outer surface of the main body portion 423 of the second band body portion 420.

As illustrated in Fig. 1, the second securing site 520 is disposed on an outer surface of the main body portion 433 of the third band body portion 430.

As illustrated in Fig. 2, the third securing site 530 is disposed on an inner surface of the main body portion 423 of the second band body portion 420.

As illustrated in Fig. 2, the fourth securing site 540 is disposed on an inner surface of the main body portion 413 of the first band body portion 410.

The first securing site 510 and the second securing site 520 are configured on a male side of a hook-and-loop fastener. The third securing site 530 and the fourth securing site 540 are configured on a female side of the hook-and-loop fastener. The hook-and-loop fastener in the present specification is a fastener that is attachable to and detachable from the surface, and is, for example, Magic Tape (registered trademark) or Velcro (registered trademark).

The second band body portion 420 and the third band body portion 430 are configured to be attachable and detachable via the third securing site 530 located on the inner surface of the main body portion 423 of the second band body portion 420 and the second securing site 520 located on the outer surface of the main body portion 433 of the third band body portion 430. In addition, the first band body portion 410 and the second band body portion 420 are configured to be attachable and detachable via the fourth securing site 540 located on the inner surface of the main body portion 413 of the first band body portion 410 and the first securing site 510 located on the outer surface of the main body portion 423 of the second band body portion 420.

Note that specific structures of the securing sites 510, 520, 530, and 540 are not limited as long as the cover member 100 can be secured to the right hand H1 by connecting the band body portions 410, 420, and 430 to each other in a state where the hemostatic device 10 is disposed on the right hand H1. For example, installation of some of the securing sites can be omitted, and positions where the securing sites are disposed on the band body portions 410, 420, and 430, and the like can be arbitrarily changed. In addition, when each of the securing sites 510, 520, and 530 is configured as a hook-and-loop fastener, the male side and the female side of the hook-and-loop fastener may be configured to be interchanged. In addition, each of the securing sites 510, 520, 530, and 540 may include, for example, a coupling mechanism or the like including a frame portion formed with a snap, a button, a clip, a protrusion, and an engaged portion in which a hole engageable with the frame portion is formed.

### <Support Member>

Figs. 8 to 15 illustrate the support member 300. In the drawings, arrows X1-X2 indicate the longitudinal direction of the support member 300 (the same direction as the longitudinal width wa direction of the inflatable member 210), arrows Y1-Y2 indicate the width direction of the support member 300 (the same direction as the lateral width wb direction of the inflatable member 210), and arrows Z1-Z2 indicate the thickness direction of the support member 300.

As illustrated in Figs. 4, 5, 8, 9, and 10, the support member 300 includes an upper end portion 301 located on the first band body portion 410 side and forming an end portion of the support member 300, a lower end portion 302 forming an end portion opposite to the upper end portion 301, and a pair of side surface portions 303 and 304 connecting the upper end portion 301 and the lower end portion 302.

As illustrated in Figs. 19 and 22, the upper end portion 301 can be disposed on the fingertip side (distal side) of the finger in a state where the hemostatic device 10 is attached to the right hand H1. The lower end portion 302 can be disposed on the forearm portion Ar side (proximal side) in a state where the hemostatic device 10 is attached to the right hand H1.

As illustrated in Fig. 8, the support member 300 is configured such that width w1 of the support member 300 decreases from upper end portion 301 toward the lower end portion 302.

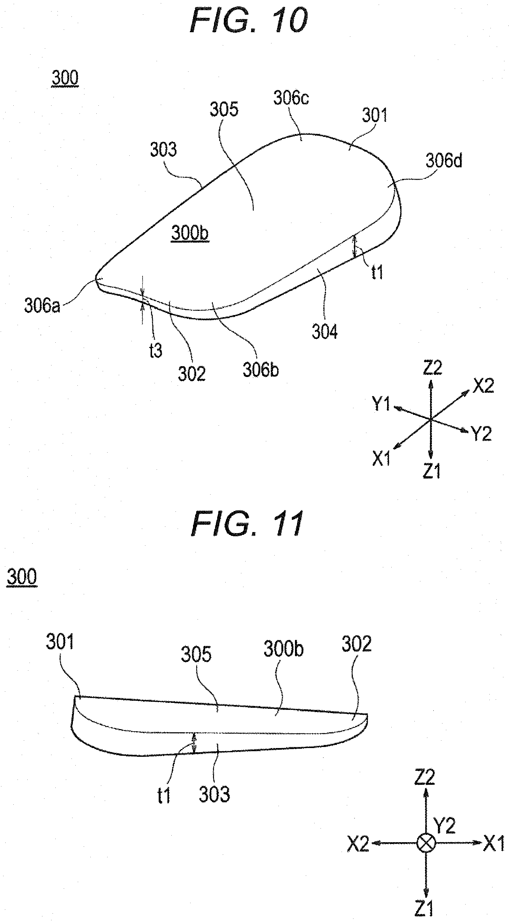

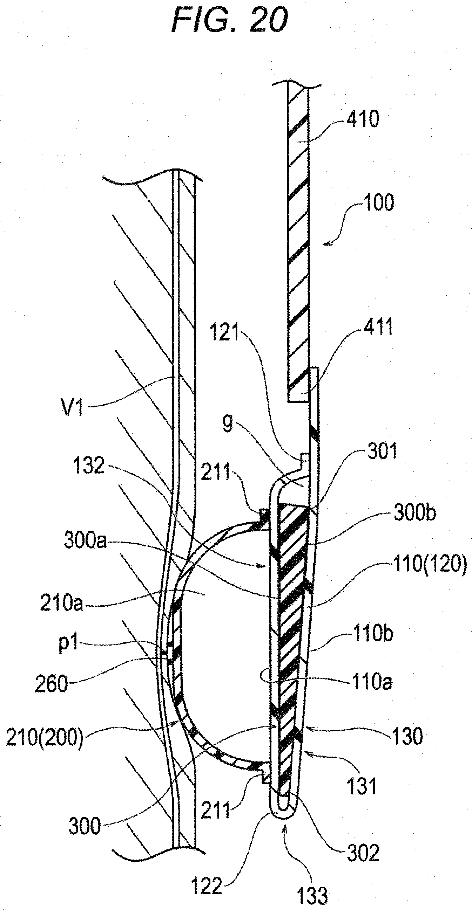

A thickness t2 (see Fig. 9) of the end surface located on the upper end portion 301 side is formed to be thicker than a thickness t3 (see Fig. 10) of the end surface located on the lower end portion 302 side. Therefore, as illustrated in Fig. 11, the support member 300 is configured such that thickness t1 of the side surface portions 303 and 304 connecting the upper end portion 301 and the lower end portion 302 decreases from the upper end portion 301 toward the lower end portion 302. That is, in the cross-sectional view illustrated in Fig. 6 and the cross-sectional view illustrated in Fig. 20, the support member 300 is inclined such that a space between an outer surface 300b and an inner surface 300a decreases from the upper end portion 301 toward the lower end portion 302.

Note that the inner surface 300a of the support member 300 is a surface disposed on the lower surface region 132 side of the holding portion 130. The outer surface 300b of the support member 300 is a surface disposed on the upper surface region 131 side of the holding portion 130 (see Fig. 6).

The lower end portion 302 of the support member 300 has a flat portion 302a having a linear shape in plan view illustrated in Fig. 8. The flat portion 302a extends substantially linearly between corner portions 306a and 306b located on the lower end portion 302 side.

The upper end portion 301 of the support member 300 has a flat portion 301a having a linear shape in plan view illustrated in Fig. 8. The flat portion 301a extends substantially linearly between corner portions 306c and 306d located on the upper end portion 301 side.

As illustrated in Figs. 7, 9, 10, 12, and 13, the outer surface 300b of the support member 300 is curved toward the second band body portion 420 and third band body portion 430 sides. That is, on the outer surface 300b of the support member 300, a top portion protruding most is located in the vicinity of the center of the support member 300 in a width direction. In addition, the top portion of the outer surface 300b of the support member 300 is located along the longitudinal direction of the support member 300. That is, the top portion of the outer surface 300b of the support member 300 is located along the longitudinal direction passing through an intermediate portion 305.

The curvature radius of the outer surface 300b of the support member 300 is substantially constant at each portion in the longitudinal direction of the support member 300.

The corner portions 306a, 306b, 306c, and 306d located at the four corners of the support member 300 are formed in a rounded curved shape.

The support member 300 has the intermediate portion 305 located between the upper end portion 301 and the lower end portion 302 in plan view illustrated in Fig. 8. The intermediate portion 305 is located in the middle in the longitudinal direction connecting the upper end portion 301 and the lower end portion 302.

The support member 300 has a bilaterally symmetrical shape relative to a center line C of the first band body portion 410 (axis along the extending direction of the first band body portion 410) in the main body portion 110. In the present embodiment, the support member 300 has a bilaterally symmetrical shape relative to a line segment that is orthogonal to an axis along a width w1' of the upper end portion 301 and passes through a point where distances from both ends of the width w1' of the upper end portion 301 are equal in plan view of Fig. 8. Therefore, the support member 300 can be disposed on the main body portion 110 so as to be bilaterally symmetrical relative to the center line C of the first band body portion 410 (axis along the extending direction of the first band body portion 410). In addition, as illustrated in plan view of Fig. 8, the side surface portions 303 and 304 are formed to be bilaterally symmetrical relative to the center line C of the first band body portion 410 in the main body portion 110. The side surface portions 303 and 304 extend bilaterally symmetrically such that the width w1 of the support member 300 decreases at a substantially constant rate from the upper end portion 301 side toward the lower end portion 302 side.

The support member 300 has a position that becomes a gravity center G1 of the outer shape of the support member 300 in plan view illustrated in Fig. 8.

The support member 300 has a substantially trapezoidal shape in which the width w1' of the upper end portion 301 is formed to be larger than a width w1" of the lower end portion 302. Therefore, the gravity center G1 of the outer shape of the support member 300 is located at a position displaced a predetermined distance from the intermediate portion 305 of the support member 300 toward the upper end portion 301 side (the lower bottom side of the trapezoidal shape).

In plan view illustrated in Fig. 8, the inflatable member 210 is disposed on the cover member 100 in a state where the center c1 of the inflatable member 210 is located on the lower end portion 302 side (the upper bottom side of the trapezoidal shape) of the support member 300 with respect to the gravity center G1 of the outer shape of the support member 300.

In the support member 300, as illustrated in Fig. 8, the width w1' of the upper end portion 301 of the support member 300 is longer than the lateral width wb of the inflatable member 210. In addition, in the support member 300, the width w1" of the lower end portion 302 of the support member 300 is shorter than the lateral width wb of the inflatable member 210.

In the present embodiment, the width w1' of the upper end portion 301 of support member 300 is defined by a dimension (maximum width) of a portion having the largest width in a region located on the upper end portion 301 side with respect to the intermediate portion 305 of the support member 300. In addition, the width w1" of the lower end portion 302 of the support member 300 is defined by a dimension of the width of the flat portion 302a located at the lower end portion 302.

As illustrated in Figs. 7, 9, 14, and 15, the inner surface 300a of the support member 300 is curved in a protruding shape in a direction away from the inflatable member 210. Similarly to the outer surface 300b, the inner surface 300a is curved toward the second band body portion 420 and third band body portion 430 sides.

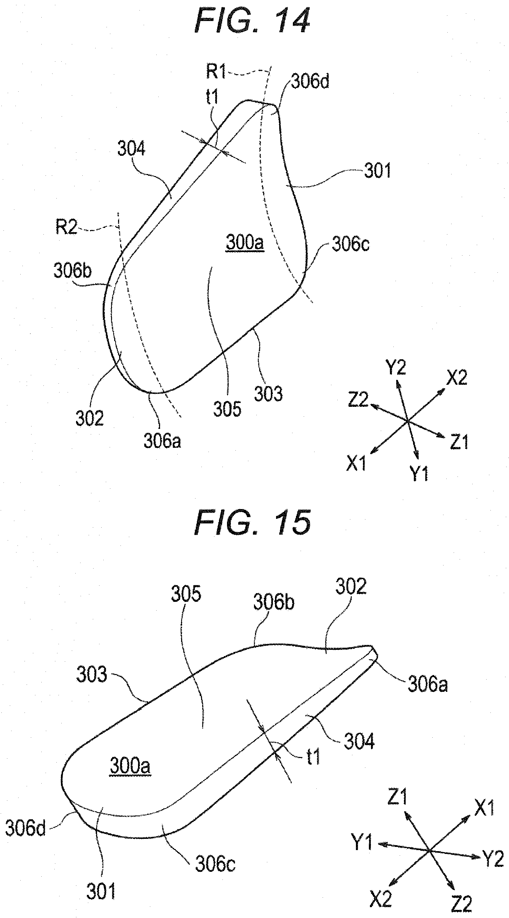

As illustrated in Fig. 14, the curvature radius of the inner surface 300a of the upper end portion 301 of the support member 300 is smaller than the curvature radius of the inner surface 300a of the lower end portion 302 of the support member 300. That is, the inner surface 300a of the support member 300 is curved more sharply at the upper end portion 301 than at the lower end portion 302. An imaginary line R1 illustrated in Fig. 14 indicates a part of an arc along the inner surface 300a of the upper end portion 301, and an imaginary line R2 indicates a part of an arc along the inner surface 300a of the lower end portion 302.

In the support member 300, the curvature radius of the inner surface 300a and the thickness t1 of the side surface portions 303 and 304 can be adjusted by adopting, for example, the manufacturing method described below.

As a constituent material of the support member 300, a curved plate-shaped member in which the curvature radius of the inner surface 300a and the outer surface 300b are substantially the same is prepared. In order to provide the above-described large-small relationship of the curvature radius between the upper end portion 301 side and the lower end portion 302 side of the inner surface 300a of the support member 300, the inner surface 300a of the plate-shaped member described above is subjected to processing of scraping the constituent material of the plate-shaped member. At this time, the amount of scraping the constituent material is increased on the lower end portion 302 side, and the amount of scraping the constituent material is gradually reduced toward the upper end portion 301 side. By processing the inner surface 300a of the plate-shaped member in this manner, the curvature radius of the inner surface 300a of the lower end portion 302 can be made larger than the curvature radius of the inner surface 300a of the upper end portion 301 (see Fig. 14). In addition, the thickness t1 of the side surface portions 303 and 304 can be configured to be reduced from the upper end portion 301 side toward the lower end portion 302 side (see Fig. 9). In addition, the outer surface 300b of the plate-shaped member is not subjected to the processing of scraping the constituent material, so that the curvature radius of the outer surface 300b of the support member 300 can be configured to maintain the curvature radius of the plate-shaped member. Accordingly, the outer surface 300b of the support member 300 can be configured to have substantially the same curvature radius at each portion in the longitudinal direction of the support member 300.

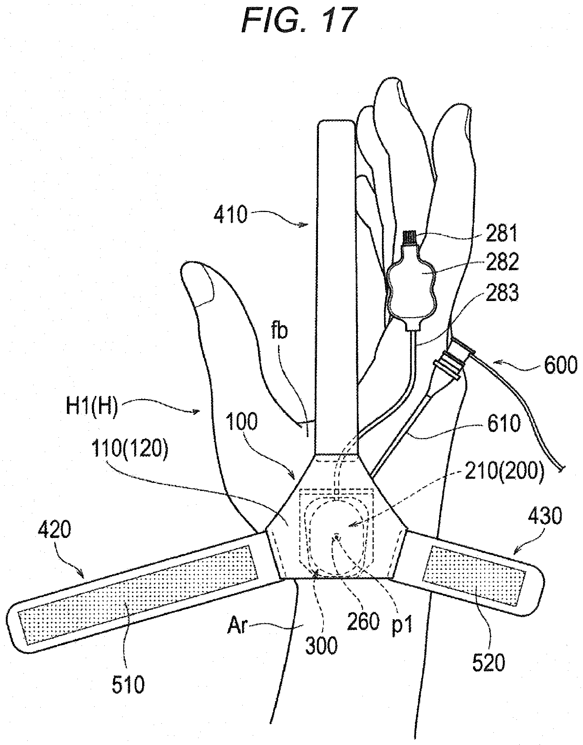

In the hemostatic device 10, a portion where the marker portion 260 of the inflatable member 210 is provided, a portion overlapping the marker portion 260 in the cover member 100 (the upper surface region 131 and the lower surface region 132 of the holding portion 130), and a portion overlapping the marker portion 260 in the support member 300 are preferably formed to be transparent. In a case where each of the members 100, 210, and 300 is configured in this manner, when attaching the hemostatic device 10 to the right hand H1 of the patient as illustrated in Figs. 17 to 19, the operator can easily visually confirm the position of the marker portion 260 disposed on the inflatable member 210 and/or the first puncture site p1 via the portion of each of the members 100, 210, and 300 that is formed to be transparent. Note that the transparency described above includes colored transparency, colorless transparency, and translucency.

The support member 300 is made of a material harder than the inflatable member 210. Therefore, in the support member 300, when the inflatable member 210 applies a compressive force to the first puncture site p1 as illustrated in Figs. 20 and 21, the support member 300 can press the inflatable member 210 to the right hand H1 of the patient. Accordingly, it is possible to prevent the inflatable member 210 from lifting from the right hand H1 of the patient.

When the inflatable member 210 is made of the above-described materials, examples of the constituent material of the support member 300 include an acrylic resin, polyvinyl chloride (in particular, hard polyvinyl chloride), polyethylene, polypropylene, polyolefin such as polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyethylene terephthalate (PET), or the like.

### <Injection Portion>

As illustrated in Figs. 1 and 2, the hemostatic device 10 includes an injection portion 281 for injecting a fluid into the inflatable member 210.

The injection portion 281 includes a connector having an incorporated check valve (not illustrated). A syringe (not illustrated) can be connected to the injection portion 281.

A buffer member 282 having an inflatable space is disposed between the injection portion 281 and the inflatable member 210. The buffer member 282 includes a flexible bag-shaped member having a space formed inside. Note that the buffer member 282 may be provided with an arrow-shaped marker indicating a direction in which the syringe is inserted into the injection portion 281.

The injection portion 281 is connected to one end side of the buffer member 282. A lumen of the injection portion 281 communicates with the space in the buffer member 282. However, when the check valve incorporated in the injection portion 281 is closed, communication between the lumen of the injection portion 281 and the space of the buffer member 282 is cut off.

The flexible tube 283 is connected to the other end side of the buffer member 282. The lumen of the tube 283 communicates with the space of the buffer member 282. In addition, an other end portion of the tube 283 that is opposite to one end portion connected to the buffer member 282 is connected to the inflatable member 210. The lumen of the tube 283 communicates with the inner cavity 210a of the inflatable member 210.

To inflate the inflatable member 210, the operator inserts a distal tubular portion of a syringe (not illustrated) into the injection portion 281 to open the check valve. The operator injects air in the syringe into the inner cavity 210a of the inflatable member 210 by pushing a plunger of the syringe in a state where the check valve of the injection portion 281 is open.

When the air is injected into the inner cavity 210a of the inflatable member 210, the inflatable member 210 is inflated. When the inflatable member 210 is inflated, the buffer member 282 communicating with the inner cavity 210a of the inflatable member 210 via the tube 283 expands. The operator can easily understand that the inflatable member 210 is inflated without leakage of air by visually confirming expansion of the buffer member 282.

To deflate the inflatable member 210, the operator inserts the distal tubular portion of the syringe to the injection portion 281 and pulls the plunger of the syringe. By performing the operation described above, the operator can discharge the air in the inner cavity 210a of the inflatable member 210 to the syringe.

Note that the injection portion 281, the buffer member 282, and the tube 283 may be prepared and provided in a state of being coupled to the inflatable member 210, or may be prepared and provided in a state of being separated from the inflatable member 210. In addition, the injection portion 281 is not particularly limited as to a specific configuration or the like as long as the supply of the fluid to the inner cavity 210a of the inflatable member 210 and the discharge of the fluid from the inner cavity 210a of the inflatable member 210 can be operated.

### <Usage Example of Hemostatic Device>

Next, a usage example and the operation and effect of the hemostatic device 10 will be described with reference to Figs. 16 to 22.

Hereinafter, a use procedure of the hemostatic device 10 when hemostasis is performed on the first puncture site p1 formed on the right hand H1 of the patient illustrated in Fig. 16 will be described.

Fig. 17 illustrates a state after performing various procedures performed using the sheath tube 610 of the introducer 600 inserted into the first puncture site p1.

When the hemostatic device 10 is attached to the right hand H1 of the patient, as illustrated in Fig. 17, the operator disposes the inflatable member 210 and the support member 300 so as to overlap the first puncture site p1. At this time, the operator can appropriately position the inflatable member 210 and the support member 300 at the first puncture site p1 by disposing the marker portion 260 at the first puncture site p1 while visually confirming the position of the marker portion 260 disposed on the inflatable member 210.

When the hemostatic device 10 is attached to the right hand H1 of the patient, the operator disposes the support member 300 so that the support member 300 overlaps the metacarpal bone B1 of the index finger and the metacarpal bone B2 of the thumb. As illustrated in Fig. 16, an interval between the metacarpal bone B1 of the index finger and the metacarpal bone B2 of the thumb gradually decreases from the fingertip side toward the forearm portion Ar side. The width w1 of the support member 300 becomes shorter from the upper end portion 301 disposed on the fingertip side toward the lower end portion 302 disposed on the forearm portion Ar side (see Fig. 8). Therefore, the operator can dispose the support member 300 along the metacarpal bone B1 of the index finger and the metacarpal bone B2 of the thumb by disposing the support member 300 to overlap the metacarpal bone B1 of the index finger and the metacarpal bone B2 of the thumb from the dorsal side of the right hand H1 of the patient. The operator can prevent a gap from being formed between the inflatable member 210 disposed on the inner surface 300a side of the support member 300 and the right hand H1 by disposing the support member 300 along the metacarpal bone B1 of the index finger and the metacarpal bone B2 of the thumb.

As illustrated in Fig. 18, the operator wraps the second band body portion 420 and the third band body portion 430 along the outer periphery of the right hand H1 of the patient. The operator can connect the second band body portion 420 and the third band body portion 430 via the securing sites 520 and 530 by bringing the third securing site 530 (see Fig. 2) disposed on the inner surface of the second band body portion 420 into contact with the second securing site 520 (see Fig. 1) disposed on the outer surface of the third band body portion 430. The operator can firmly wrap the band body portions 420 and 430 along the outer periphery of the right hand H1 by connecting the band body portions 420 and 430 to each other in a state where the support member 300 overlaps the metacarpal bone B1 of the index finger and the metacarpal bone B2 of the thumb.

The outer surface 300b of the support member 300 is curved toward the second band body portion 420 side (side surface portion 303 side) and the third band body portion 430 side (side surface portion 304 side) (see Figs. 5, 9, and 10). Therefore, as illustrated in Fig. 18, by wrapping the second band body portion 420 and the third band body portion 430 along the right hand H1 of the patient, the support member 300 can be pressed to the right hand H1 from the outer surface 300b side along the outer periphery of the right hand H1 as illustrated in Fig. 21. Therefore, the hemostatic device 10 can increase the securing force of the support member 300 and the inflatable member 210 to the right hand H1 of the patient.

As illustrated in Fig. 19, the operator passes the first band body portion 410 through the inter-finger portion fb located between the thumb and the index finger of the right hand H1 of the patient, and disposes a part of the first band body portion 410 on the palm side of the right hand H1 of the patient. At this time, the operator can connect the first band body portion 410 and the second band body portion 420 via the securing sites 510 and 540 by bringing the fourth securing site 540 (see Fig. 2) disposed on the inner surface of the first band body portion 410 into contact with the first securing site 510 (see Fig. 1) disposed on the outer surface of the second band body portion 420.

The support member 300 includes the upper end portion 301 and the side surface portions 303 and 304. The first band body portion 410 extends from the upper end portion 301 side, the second band body portion 420 extends from the side surface portion 303 side, and the third band body portion 430 extends from the side surface portion 304 side (see Fig. 5). Therefore, the hemostatic device 10 can secure the support member 300 to the right hand H1 of the patient in a state in which a uniform force is applied to the position corresponding to each portion 301, 303, and 304 of the support member 300 by connecting the band body portions 410, 420, and 430 to each other.

The width w1' of the upper end portion 301 of the support member 300 is longer than the lateral width wb of the inflatable member 210, and the width w1" of the lower end portion 302 of the support member 300 is shorter than the lateral width wb of the inflatable member 210 (see Fig. 8). For example, when the first band body portion is firmly tightened to the inter-finger portion fb in a state where the support member is disposed on the right hand H1 of the patient, the inflatable member may be displaced to the lower end portion side (forearm portion Ar side) of the support member that is located on the side opposite to the side where the first band body portion is tightened. To solve such a problem, in the support member 300 of the present embodiment, the width w1" of the lower end portion 302 of the support member 300 is shorter than the lateral width wb of the inflatable member 210 as described above, and it is possible to suppress that the inflatable member 210 is displaced to the lower end portion 302 side of the support member 300. In particular, the support member 300 is curved in a protruding shape such that the inner surface 300a of the support member 300 is away from the inflatable member 210 (see Figs. 14 and 21). Therefore, the support member 300 can press both end portions in a lateral width direction of the inflatable member 210 at positions of both end portions in the width direction of the inner surface 300a of the support member 300. Therefore, the hemostatic device 10 can effectively prevent the inflatable member 210 from being displaced toward the lower end portion 302 side of the support member 300 when the inflatable member 210 is inflated.

The support member 300 is bilaterally symmetrical relative to the center line C of the first band body portion 410 in the main body portion 110 (see Figs. 5 and 8). Therefore, the hemostatic device 10 can uniformly apply a force along a bilaterally symmetrical direction to the support member 300 by wrapping the band body portions 420 and 430 bilaterally symmetrically extending based on the first band body portion 410 around the right hand H1 of the patient. Accordingly, the hemostatic device 10 can prevent displacement of the support member 300 in a state where the support member 300 is disposed on the right hand H1 of the patient.

The inner surface 300a of the support member 300 is curved in a protruding shape in a direction away from the inflatable member 210 (see Figs. 7 and 21). An inclined portion is present in each portion (for example, the vicinity of the snuff box Sb) of the right hand H1 of the patient. In addition, the shape of the inclined portion of the right hand H1 depends on individual differences of the patient. For example, when the inner surface of the support member is formed in a flat shape, it is difficult to dispose the support member along the inclined portion of the right hand H1. The inner surface 300a of the support member 300 of the hemostatic device 10 has a shape curved in a protruding shape in a direction away from the inflatable member 210 as described above. Therefore, when the inner surface 300a of the support member 300 is disposed so as to overlap the inclined portion of the right hand H1 of the patient, the inner surface 300a of the support member 300 can be disposed along the right hand H1 of the patient. Accordingly, the hemostatic device 10 can dispose the support member 300 to overlap the first puncture site p1 without depending on the dominant hand of the patient or the way of placing by the operator. Therefore, when the hemostatic device 10 performs hemostatic compression at the first puncture site p1 using the inflatable member 210, the support member 300 can be suitably disposed at a desired position in the vicinity of the first puncture site p1.

Further, since the inner surface 300a of the support member 300 is disposed along the body surface of the right hand H1 when the operator wraps the second band body portion 420 and the third band body portion 430 along the right hand H1 of the patient, the hemostatic device 10 can be disposed such that each of the band body portions 420 and 430 extending from each of the side surface portions 303 and 304 of the support member 300 approaches or adheres to the body surface of the right hand H1 of the patient (see Fig. 21). Therefore, the hemostatic device 10 can increase the securing force of the support member 300 and the inflatable member 210 to the right hand H1 of the patient. Therefore, in a state where the hemostatic device 10 is attached to the right hand H1 of the patient, the support member 300 and the inflatable member 210 are less likely to be displaced even when an impact or the like is applied from the outside.

The operator inflates the inflatable member 210 by injecting air into the inflatable member 210 in a state where the syringe is connected to the injection portion 281. In the hemostatic device 10, as illustrated in Figs. 20 and 21, when the inflatable member 210 is inflated, the inflatable member 210 applies a compressive force to the first puncture site p1.

When the inflatable member 210 is inflated in a state where the hemostatic device 10 is attached to the right hand H1 of the patient, the support member 300 presses the inflatable member 210 to the right hand H1 of the patient. Accordingly, the hemostatic device 10 can prevent the inflatable member 210 from lifting from the right hand H1 of the patient.

When performing hemostasis using the hemostatic device, the operator can more strongly tighten, for example, the first band body portion disposed at the inter-finger portion fb so as to increase the securing force of the support member with respect to the right hand H1 of the patient. By strongly tightening the first band body portion, the operator can firmly secure the support member to the right hand H1 of the patient even when the support member is disposed in an unstable state on the right hand H1 of the patient. However, when the first band body portion is strongly tightened, the patient may feel pain. Therefore, when the first band body portion cannot be strongly tightened due to pain or the like of the patient, the hemostatic device may be disposed such that the inner surface of the support member is inclined with respect to the body surface of the right hand H1 of the patient. Specifically, the upper end portion of the support member on which the first band body portion is disposed is more excessively lifted to a position away from the body surface of the right hand of the patient than the lower end portion located on the opposite side of the upper end portion. Accordingly, in the hemostatic device, the support member is disposed in an inclined manner such that the distance between the inner surface of the support member and the body surface of the right hand H1 of the patient gradually decreases from the upper end portion toward the lower end portion of the support member.

In the hemostatic device 10, the thickness t1 of the side surface portions 303 and 304 of the support member 300 is configured to decrease from the upper end portion 301 toward the lower end portion 302. Additionally, the first band body portion 410 configured to be disposed at the inter-finger portion fb located between the fingers of the patient extends from the upper end portion 301 side of the support member 300 and is configured to press the upper end portion 301 side of the support member 300 to the right hand H1 of the patient. In the hemostatic device 10 configured as described above, when the inflatable member 210 starts inflating in a state where the support member 300 and the inflatable member 210 are secured to the right hand H1 of the patient, the inflatable member 210 applies a force to vicinity of the upper end portion 301 of the support member 300. The upper end portion 301 side of the support member 300 is lifted by the force applied by the inflatable member 210. Accordingly, as illustrated in Fig. 20, when the inflatable member 210 is in an inflated state, the support member 300 is disposed such that the inner surface 300a of the support member 300 is substantially parallel to the body surface of the right hand H1 of the patient. Therefore, when the inflatable member 210 is inflated, the hemostatic device 10 can apply a compressive force to the first puncture site p1 in a vertical direction.

The inner surface 300a of the support member 300 is curved in a protruding shape in a direction away from the inflatable member 210 (see Figs. 7 and 21). Therefore, as illustrated in Fig. 21, the hemostatic device 10 is disposed such that the support member 300 is along the body surface of the right hand H1 of the patient in a state where the inflatable member 210 is inflated. When the inflatable member 210 is inflated, the hemostatic device 10 presses the inflatable member 210 along the body surface of the right hand H1 of the patient using the inner surface 300a of the support member 300. Accordingly, in the hemostatic device 10, the inflatable member 210 applies a compressive force in a direction toward the first puncture site p1 from the center side in the width direction of the inner surface 300a of the support member 300 and the side surface portions 303 and 304 located at both end portions in the width direction of the inner surface 300a of the support member 300. Therefore, the hemostatic device 10 can effectively increase the compressive force applied to the first puncture site p1 using the inflatable member 210 as compared with the case where the inner surface 300a of the support member 300 is formed in a flat surface shape.

The outer surface 300b of the support member 300 is curved toward the second band body portion 420 side (side surface portion 303 side) and the third band body portion 430 side (side surface portion 304 side) (see Figs. 5, 9, and 10). Therefore, as illustrated in Fig. 21, in a state where the hemostatic device 10 is attached to the right hand H1 of the patient and the inflatable member 210 is inflated, the outer surface 110b of the main body portion 110 located on the outer surface 300b side of the support member 300 has a curved shape along the outer surface 300b of the support member 300. In the hemostatic device 10, since the outer surface 300b of the support member 300 and the main body portion 110 have a curved shape in a state where the inflatable member 210 is inflated, when an impact or the like is applied from the outer surface 110b of the main body portion 110 due to contact or the like of the hemostatic device 10 with a surrounding article, the impact can be diverted to the surrounding along the curved surfaces 110b and 300b.

Therefore, in a state where the hemostatic device 10 is attached to the patient, it is possible to effectively prevent the occurrence of the displacement of the support member 300 and the inflatable member 210 even when an impact is applied from the outside.

Note that in order to exhibit the effect described above, the hemostatic device 10 is preferably disposed such that the entire inflatable member 210 is accommodated inside the support member 300 in the plane direction in plan view illustrated in Fig. 8. In the hemostatic device 10, when the inflatable member 210 is disposed in the manner described above, the entire inflatable member 210 is covered with the support member 300, so that it is possible to effectively prevent the impact applied from the outer surface 110b of the main body portion 110 from being directly transmitted to the inflatable member 210.

Fig. 22 illustrates a state when the patient to which the hemostatic device 10 is attached twists the wrist toward the dorsal side of the right hand H1. In the hemostatic device 10, the lower end portion 302 of the support member 300 is disposed on the forearm portion Ar side in a state where the hemostatic device 10 is attached to the right hand H1 of the patient. The lower end portion 302 of the support member 300 has the flat portion 302a having a linear shape (see Fig. 8). Therefore, as illustrated in Fig. 22, when the patient performs a movement of twisting the wrist toward the dorsal side of the right hand H1, the flat portion 302a located at the lower end portion 302 can prevent the support member 300 from biting into the body surface such as the wrist or the forearm portion Ar of the patient. Therefore, the hemostatic device 10 can prevent the support member 300 from biting into the body surface when the patient performs a movement such as twisting the wrist during hemostasis at the first puncture site p1 formed on the right hand H1 of the patient. For example, in a case where the support member is formed in a triangular shape or the like in plan view and a corner portion (vertex) is disposed on the forearm portion Ar side, when the patient performs a movement of twisting the wrist, the corner portion located at the lower end portion bits into the body surface of the patient, and the patient may feel pain.

The support member 300 has a position that becomes a gravity center G1 of the outer shape of the support member 300 in plan view illustrated in Fig. 8. The gravity center G1 is located on the upper end portion 301 side with respect to the intermediate portion 305 located between the upper end portion 301 and the lower end portion 302 of the support member 300. In addition, the inflatable member 210 is disposed on the cover member 100 in a state where the center c1 of the inflatable member 210 is located on the lower end portion 302 side of the support member 300 with respect to the gravity center G1 of the support member 300. The hemostatic device 10 is configured such that the inflatable member 210 inflates in a state where the center c1 of the inflatable member 210 is located on the lower end portion 302 side of the support member 300 with respect to the gravity center G1 of the support member 300. In the support member 300, since the gravity center G1 of the support member 300 is located on the upper end portion 301 side with respect to the center c1 of the inflatable member 210, when the inflatable member 210 is inflated, a part of the inflatable member 210 located on the upper end portion 301 side of the support member 300 can be pressed to the body surface of the right hand H1 of the patient. Therefore, when the inflatable member 210 is inflated, the hemostatic device 10 can lift the lower end portion 302 side of the support member 300 from the body surface of the right hand H1 of the patient. Therefore, the hemostatic device 10 can effectively prevent the lower end portion 302 of the support member 300 disposed on the wrist or forearm portion Ar side of the patient from biting into the body surface while the inflatable member 210 inflated in a state where the hemostatic device 10 is attached to the right hand H1 of the patient applies a compressive force to the first puncture site p1.

In addition, each of the corner portions 306a, 306b, 306c, and 306d of the support member 300 has a rounded shape (see Fig. 8). Therefore, when the inflatable member 210 is inflated to apply a compressive force to the first puncture site p1, the hemostatic device 10 can prevent the corner portions 306a, 306b, 306c, and 306d of the support member 300 from biting into the body surface of the right hand H1 of the patient.

As illustrated in Fig. 8, since the hemostatic device 10 has a substantially trapezoidal shape in which the width w1 of the support member 300 gradually decreases from the upper end portion 301 side toward the lower end portion 302 side, the effects described below are provided.

When a general trapezoid formed to have substantially the same length in the longitudinal direction is compared with a triangle, a distance between an upper end portion corresponding to a base or a lower base and the gravity center is larger in the trapezoid. Therefore, when the hemostatic device 10 is configured to use the inflatable member 210 having a substantially oval shape as illustrated in Fig. 8 and to be disposed to overlap the center c1 of the inflatable member 210 with the center in the longitudinal direction of the support member 300, the distance between the gravity center G1 and the gravity center of the inflatable member 210 can be reduced by comparing the case where the support member 300 is formed to be a trapezoid and the case where the support member 300 is formed to be a triangle. Since the distance between the gravity center G1 of the support member 300 and the gravity center of the inflatable member 210 is small, the hemostatic device 10 can suppress the inclination of the support member 300 due to excessive lifting of the lower end portion 302 side of the support member 300 when the inflatable member 210 is inflated, and can suitably apply a compressive force to the first puncture site p1. Therefore, in the hemostatic device 10, the inflatable member 210 can be firmly pressed to the right hand H1 by the support member 300, and the compressive force applied to the first puncture site p1 by the inflatable member 210 can be effectively increased. Note that the gravity center of the inflatable member 210 in the description described above means the gravity center of a constituent portion on the bottom surface side of the inflatable member 210 (a region where the inflatable member 210 is projected in plan view on the inner surface 110a of the main body portion 110). The gravity center of the inflatable member 210 formed in a substantially oval shape as in the present embodiment is located at a position overlapping the center c1 of the inflatable member 210 in plan view illustrated in Fig. 8 (see Fig. 8).

In addition, the hemostatic device 10 is preferably disposed such that the entire inflatable member 210 is disposed inside the support member 300 in plan view illustrated in Fig. 8. Since the inflatable member 210 is disposed in the manner described above, the support member 300 can firmly press the inflatable member 210, and the compressive force applied to the first puncture site p1 by the inflatable member 210 can be effectively increased.

In addition, the hemostatic device 10 is configured to have a substantially trapezoidal shape in which the width w1 of the support member 300 gradually decreases from the upper end portion 301 side toward the lower end portion 302 side. Therefore, the entire inflatable member 210 can be disposed inside the support member 300, and the positional relationship between the gravity center G1 of the support member 300 and the center c1 of the inflatable member 210 described above can be suitably maintained.

For example, in a case where the support member is formed as a triangle in plan view and the vertex of the triangle is disposed on the forearm portion Ar side, when the entire inflatable member is disposed inside the support member while ensuring the longitudinal width and the lateral width of the inflatable member as large as possible, the hemostatic device needs to dispose the inflatable member also in the vicinity of the vertex of the triangle of the support member. At this time, the inflatable member is formed in an elongated shape along the longitudinal width direction in plan view. When the inflatable member is formed in the manner described above, the inflatable member has to be formed to be smaller in lateral width than the inflatable member 210 of the present embodiment having a shape corresponding to the support member formed in a substantially trapezoidal shape. Therefore, in the inflatable member formed corresponding to the support member formed as a triangle in plan view, the compression area where the inflatable member applies the compressive force to the vicinity of the first puncture site p1 is reduced. Accordingly, it is difficult for the hemostatic device to effectively apply the compressive force to the first puncture site p1. Further, in a case where the support member is formed as a triangle as described above and the inflatable member 210 is formed to be elongated along the longitudinal direction of the support member so that the lateral width becomes narrow, it becomes difficult to set the positional relationship between the gravity center G1 of the support member 300 and the center c1 of the inflatable member 210 described above (see Fig. 8). In addition, when the inflatable member cannot be disposed in the vicinity of the vertex of the triangle, the inflatable member cannot be disposed between the support member and the body surface, and therefore, when the patient performs a movement such as twisting the wrist (see Fig. 22), the lower end portion of the support member corresponding to the vertex of the triangle tends to bit into the body surface of the patient.

After inflating the inflatable member 210, the operator removes the sheath tube 610 of the introducer 600 from the first puncture site p1 as illustrated in Fig. 19. The operator confirms that there is no bleeding from the first puncture site p1 while performing hemostasis using the hemostatic device 10. In a case where there is bleeding from the first puncture site p1, the operator can adjust the injection amount of air into the inflatable member 210.

According to the above procedure, the operator can perform hemostasis at the first puncture site p1 formed on the right hand H1 of the patient using the hemostatic device 10.

The operator can also use the hemostatic device 10 for hemostasis at the second puncture site p2 illustrated in Fig. 16, for example. When hemostasis is performed at the second puncture site p2, the operator disposes the support member 300 and the inflatable member 210 so as to overlap the second puncture site p2, similarly to the case of hemostasis at the first puncture site p1.

In the hemostatic device 10, the curvature radius of the inner surface 300a of the upper end portion 301 of the support member 300 is smaller than the curvature radius of the inner surface 300a of the lower end portion 302 of the support member 300 (see Fig. 14). As described above, the vicinity of the snuff box where the support member 300 and the inflatable member 210 are disposed is surrounded by the extensor pollicis longus muscle tendon T1 and the extensor pollicis brevis muscle tendon T2 (see Fig. 16). In addition, the distance between the extensor pollicis longus muscle tendon T1 and the extensor pollicis brevis muscle tendon T2 narrows toward the forearm portion Ar side. In the support member 300, the curvature radius of the inner surface 300a of the upper end portion 301 disposed at the fingertip side position of the extensor pollicis longus muscle tendon T1 and the extensor pollicis brevis muscle tendon T2 is small, and the curvature radius of the inner surface 300a of the lower end portion 302 disposed at the forearm portion Ar side position of the extensor pollicis longus muscle tendon T1 and the extensor pollicis brevis muscle tendon T2 is large so as to correspond to a change in distance between the extensor pollicis longus muscle tendon T1 and the extensor pollicis brevis muscle tendon T2. Therefore, when the support member 300 is disposed on the right hand H1 of the patient for hemostasis at the second puncture site p2, the hemostatic device 10 is disposed such that the support member 300 is along the extensor pollicis longus muscle tendon T1 and the extensor pollicis brevis muscle tendon T2. As a result, the hemostatic device 10 can firmly secure the support member 300 to the vicinity of the second puncture site p2 located in the vicinity of the snuff box Sb at the time of hemostasis at the second puncture site p2.

As described above, the hemostatic device 10 according to the present embodiment includes the cover member 100 configured to cover the first puncture site p1, and the pressing member 200 connected to the cover member 100 and configured to compress the first puncture site p1. The cover member 100 includes the main body portion 110 where the pressing member 200 is located, the first band body portion 410 that is configured to be disposed between fingers of the patient and extends from the main body portion 110 in the first direction, the second band body portion 420 that extends from the main body portion 110 in the second direction different from the first direction, and the third band body portion 430 that faces the second band body portion 420 across the pressing member 200 and extends from the main body portion 110 in the third direction different from the first direction and the second direction. The main body portion 110 includes the support member 300 formed of a material harder than the pressing member 200 and configured to overlap the pressing member 200. The support member 300 includes the upper end portion 301 located on the first band body portion 410 side and forming an end portion of the support member 300, the lower end portion 302 forming an end portion opposite to the upper end portion 301, and the side surface portions 303 and 304 connecting the upper end portion 301 and the lower end portion 302. The support member 300 is configured such that width w1 of the support member 300 decreases from the upper end portion 301 toward the lower end portion 302, and the thickness t1 of the side surface portions 303 and 304 decreases from the upper end portion 301 toward the lower end portion 302. The lower end portion 302 has the flat portion 302a having a linear shape in plan view, and the outer surface 300b of the support member 300 is curved toward the second band body portion 420 side and the third band body portion 430 side.

With the hemostatic device 10 configured as described above, the support member 300 made of a material harder than the pressing member 200 is disposed so as to cover the first puncture site p1 together with the pressing member 200 when performing hemostasis at the first puncture site p1 formed at a predetermined position avoiding the metacarpal bone B1 of the index finger of the right hand H1 and the metacarpal bone B2 of the thumb of the right hand H1. The support member 300 can prevent the pressing member 200 from lifting from the right hand H1 of the patient by pressing the pressing member 200 to the right hand H1. In addition, the width w1 of the support member 300 becomes shorter from the upper end portion 301 toward the lower end portion 302. Therefore, when the support member 300 is disposed on the right hand H1, the support member 300 can be disposed along the metacarpal bone B1 of the index finger and the metacarpal bone B2 of the thumb. Therefore, in the hemostatic device 10 described above, the second band body portion 420 and the third band body portion 430 can be firmly wrapped along the outer periphery of the right hand H1. In addition, in the support member 300, the thickness t1 of the side surface portions 303 and 304 becomes thinner from the upper end portion 301 toward the lower end portion 302. Additionally, the first band body portion 410 configured to be disposed between fingers of the patient is configured to extend from the upper end portion 301 side of the support member 300 and press the upper end portion 301 side of the support member 300 to the right hand H1. Therefore, the pressing member 200 can appropriately apply a force to the upper end portion 301 side of the support member 300 when hemostasis starts in a state where the hemostatic device 10 is attached to the right hand H1. The support member 300 lifts the upper end portion 301 side of the support member 300 using the force applied by the pressing member 200. Accordingly, the hemostatic device 10 can dispose the support member 300 such that the inner surface 300a of the support member 300 is substantially parallel to the body surface of the right hand H1. Therefore, in the hemostatic device 10, the compressive force of the pressing member 200 can be directed in a direction perpendicular to the first puncture site p1 by securing each of the band body portions 410, 420, and 430 to the right hand H1 in a state where the pressing member 200 and the support member 300 are disposed at the first puncture site p1. Accordingly, the hemostatic device 10 can effectively apply the compressive force to the first puncture site p1.

Further, in the hemostatic device 10, the lower end portion 302 of the support member 300 has the flat portion 302a having a linear shape in plan view. The flat portion 302a having a linear shape located at the lower end portion 302 of the support member 300 prevents the lower end portion 302 of the support member 300 from biting into the body surface such as the wrist or the forearm portion Ar when the patient performs a movement such as twisting the wrist toward the dorsal side of the right hand H1 in a state where the hemostatic device 10 is attached to the right hand H1. Therefore, the hemostatic device 10 described above can suppress the patient from feeling pain even when the patient moves the wrist or the like while performing the hemostatic compression on the first puncture site p1 using the pressing member 200.

In addition, in the hemostatic device 10, the outer surface 300b of the support member 300 is curved toward the second band body portion 420 side and the third band body portion 430 side. Therefore, in the hemostatic device 10 described above, when the operator wraps the second band body portion 420 and the third band body portion 430 connected to the main body portion 110 of the cover member 100 along the outer periphery of the right hand H1, the second band body portion 420 and the third band body portion 430 are wrapped along the curved outer surface 300b of the support member 300. Accordingly, the hemostatic device 10 can increase the securing force of the support member 300 and the pressing member 200 to the right hand H1.

In addition, the support member 300 has a position that becomes the gravity center G1 of the outer shape of the support member 300 in plan view. The gravity center G1 is located on the upper end portion 301 side with respect to the intermediate portion 305 located between the upper end portion 301 and the lower end portion 302. The pressing member 200 is disposed on the cover member 100 in a state where the center c1 of the pressing member 200 is located on the lower end portion 302 side with respect to the gravity center G1.

With the hemostatic device 10 configured as described above, when the inflatable member 210 is inflated, the support member 300 is lifted from the body surface of the right hand H1 of the patient on the lower end portion 302 side where the center c1 of the inflatable member 210 is disposed with respect to the upper end portion 301 side where the gravity center G1 is located as a result of the inflation of the inflatable member 210. Therefore, the hemostatic device 10 can prevent the lower end portion 302 of the support member 300 disposed on the wrist or forearm portion Ar side of the patient from biting into the body surface while the inflatable member 210 inflated in a state where the hemostatic device 10 is attached to the right hand H1 of the patient applies a compressive force to the first puncture site p1.

The support member 300 is bilaterally symmetrical relative to the center line C of the first band body portion 410. The inner surface 300a of the support member 300 is curved in a protruding shape in a direction away from the pressing member 200.

Therefore, with the hemostatic device 10 configured as described above, since the support member 300 is bilaterally symmetrical relative to the center line C of the first band body portion 410 in the main body portion 110, the hemostatic device 10 can uniformly apply a force along a bilaterally symmetrical direction to the support member 300 by wrapping the band body portions 420 and 430 bilaterally symmetrically extending based on the first band body portion 410 around the right hand H1 of the patient. Accordingly, the hemostatic device 10 can prevent displacement of the support member 300 in a state where the support member 300 is disposed on the right hand H1 of the patient.

In addition, with the hemostatic device 10 configured as described above, since the inner surface 300a of the support member 300 is curved in a protruding shape in a direction away from the pressing member 200, when the inner surface 300a of the support member 300 is disposed to overlap the inclined portion of the right hand H1 of the patient, the inner surface 300a of the support member 300 can be disposed along the right hand H1 of the patient. Accordingly, the hemostatic device 10 can dispose the support member 300 to overlap the first puncture site p1 without depending on the dominant hand of the patient or the way of placing by the operator. Therefore, when the hemostatic device 10 performs hemostatic compression at the first puncture site p1 using the inflatable member 210, the support member 300 can be suitably disposed at a desired position in the vicinity of the first puncture site p1.

In addition, with the hemostatic device 10 configured as described above, since the inner surface 300a of the support member 300 is curved in a protruding shape in a direction away from the pressing member 200, the inner surface 300a of the support member 300 can be disposed along the body surface of the right hand H1 of the patient. when the inflatable member 210 is inflated, the hemostatic device 10 presses the inflatable member 210 along the body surface of the right hand H1 of the patient using the inner surface 300a of the support member 300. Accordingly, in the hemostatic device 10, the inflatable member 210 applies a compressive force in a direction toward the first puncture site p1 from the center side in the width direction of the inner surface 300a of the support member 300 and the side surface portions 303 and 304 located at both end portions in the width direction of the inner surface 300a of the support member 300. Therefore, the hemostatic device 10 can effectively increase the compressive force applied to the first puncture site p1 using the inflatable member 210 as compared with the case where the inner surface 300a of the support member 300 is formed in a flat surface shape.

The curvature radius of the inner surface 300a of the upper end portion 301 of the support member 300 is smaller than the curvature radius of the inner surface 300a of the lower end portion 302 of the support member 300.

With the hemostatic device 10 configured as described above, in the support member 300, the curvature radius of the inner surface 300a of the upper end portion 301 disposed at the fingertip side position of the extensor pollicis longus muscle tendon T1 and the extensor pollicis brevis muscle tendon T2 is small, and the curvature radius of the inner surface 300a of the lower end portion 302 disposed at the forearm portion Ar side position of the extensor pollicis longus muscle tendon T1 and the extensor pollicis brevis muscle tendon T2 is large so as to correspond to a change in distance between the extensor pollicis longus muscle tendon T1 and the extensor pollicis brevis muscle tendon T2. Therefore, when the support member 300 is disposed on the right hand H1 of the patient for hemostasis at the second puncture site p2, the hemostatic device 10 is disposed such that the support member 300 is along the extensor pollicis longus muscle tendon T1 and the extensor pollicis brevis muscle tendon T2. As a result, the hemostatic device 10 can firmly secure the support member 300 to the vicinity of the second puncture site p2 located in the vicinity of the snuff box Sb at the time of hemostasis at the second puncture site p2.

The pressing member 200 is the inflatable member 210 that can be inflated by injection of a fluid. The inflatable member 210 has a longitudinal width wa passing through a center c1 of the inflatable member 210 and a lateral width wb orthogonal to the longitudinal width wa and passing through the center c1 of the inflatable member 210 in a state where the inflatable member 210 is deflated. The lateral width wb is shorter than the longitudinal width wa. The width w1' of the upper end portion 301 of the support member 300 is longer than the lateral width wb of the inflatable member 210, and the width w1" of the lower end portion 302 is shorter than the lateral width wb of the inflatable member 210.

With the hemostatic device 10 configured as described above, the width w1' of the upper end portion 301 of the support member 300 is longer than the lateral width wb of the inflatable member 210, and the width w1" of the lower end portion 302 of the support member 300 is shorter than the lateral width wb of the inflatable member 210, and therefore, when the inflatable member 210 disposed at the first puncture site p1 is inflated, the inflatable member 210 is supported by both end portions in the width direction of the lower end portion 302 of the support member 300. Therefore, the hemostatic device 10 can suppress the inflatable member 210 from being displaced toward the lower end portion 302 side of the support member 300 when the inflatable member 210 is inflated.

### <Modification>

Next, a modification of the embodiment described above will be described. In the description of the modification, the descriptions of the members, the use procedure of the hemostatic device, and the like already described in the above-described embodiment will be appropriately omitted. In addition, contents that are not particularly described in the modification can be the same as those in the above-described embodiment.

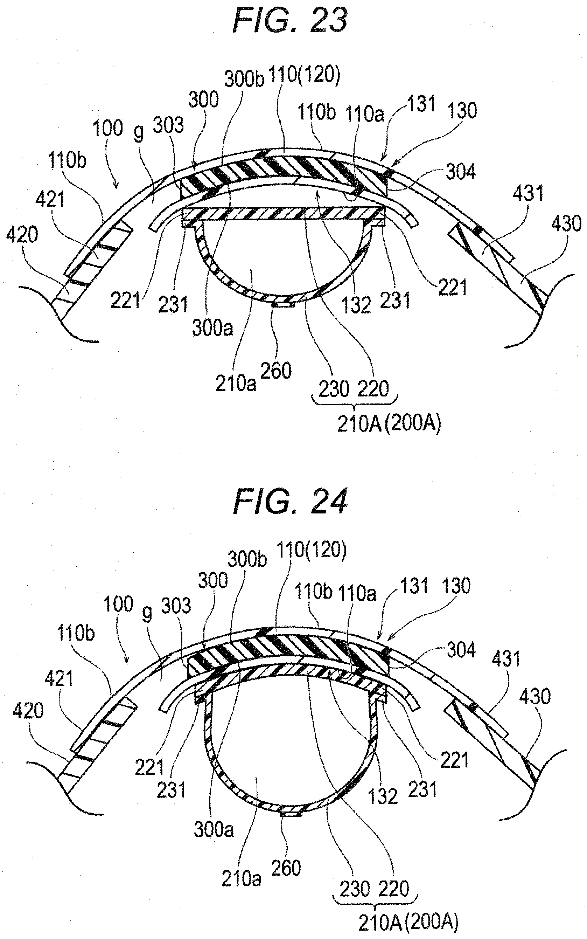

The hemostatic device according to the modification is different from the hemostatic device 10 according to the above-described embodiment in the structure of a pressing member 200A (inflatable member 210A). In the above-described embodiment, the inflatable member 210 includes one sheet material connected to the main body portion 110 of the cover member 100 (see Figs. 6 and 7). In the present modification, as illustrated in Figs. 23 and 24, the inflatable member 210A is configured by connecting an edge portion 221 of a first sheet material 220 and an edge portion 231 of a second sheet material 230. The thickness of the first sheet material 220 is configured to be larger than the thickness of the second sheet material 230.

Figs. 23 and 24 are partial cross-sectional views of the hemostatic device according to the modification. Fig. 23 illustrates a state before the inflatable member 210A is inflated, and Fig. 24 illustrates a state in which the inflatable member 210A is inflated. Figs. 23 and 24 are cross-sectional views corresponding to Fig. 7 of the above-described embodiment.

As illustrated in Figs. 23 and 24, the pressing member 200A includes the inflatable member 210A that is inflatable by injection of a fluid.

The inflatable member 210A is formed by connecting the edge portion 221 located at the outer peripheral edge of the first sheet material 220 and the edge portion 231 located at the outer peripheral edge of the second sheet material 230. The inner cavity 210a is formed between the first sheet material 220 and the second sheet material 230. The edge portion 221 of the first sheet material 220 and the edge portion 231 of the second sheet material 230 can be connected by, for example, bonding or fusing.

The first sheet material 220 is connected to the cover member 100 at a position overlapping the support member 300. The inflatable member 210A is secured to the cover member 100 by connecting the first sheet material 220 to a part of the cover member 100 (the surface of the holding portion 130). In the present modification, the first sheet material 220 is secured to the cover member 100 by connecting a part of the edge portion 221 of the first sheet material 220 to the holding portion 130. Note that, as illustrated in Fig. 23, the first sheet material 220 and the holding portion 130 are connected at two opposing positions.

The edge portion 231 of the second sheet material 230 is located at the edge portion (end portion in the lateral width wb direction located on each side surface portions 303 and 304 side of the support member 300) of the support member 300. By disposing the edge portion 231 of the second sheet material 230 in this manner, the edge portion of the inflatable member 210A is supported by the edge portion of the support member 300. Note that, in the present modification, the edge portion 221 of the first sheet material 220 and the edge portion 231 of the second sheet material 230 are located at the edge portion (end portion in the lateral width wb direction located on each side surface portions 303 and 304 side of the support member 300) of the support member 300.

As illustrated in Fig. 24, the inflatable member 210A is configured such that a portion including the second sheet material 230 has a larger inflation deformation amount than a portion including the first sheet material 220 in a state where the inflatable member 210A is inflated. In the present embodiment, the thickness of the second sheet material 230 is thinner than the thickness of the first sheet material 220. Therefore, when a fluid such as air is injected into the inner cavity 210a, the portion including the second sheet material 230 inflates and deforms more than the portion including the first sheet material 220 in the inflatable member 210A.

In a state where the inflatable member 210A illustrated in Fig. 23 is deflated, a position where the first sheet material 220 and the second sheet material 230 are connected (position where the edge portions 221 and 231 are connected) in an up-down direction in the drawing, which is the inflation direction, is set at a position deviated on the support member 300 side with respect to the center position of the entire inflatable member 210A.

As illustrated in Fig. 24, when the inflatable member 210A is inflated, the edge portion 231 (in the present modification, the edge portions 221 and 231 of the sheet materials 220 and 230) of the second sheet material 230 are pressed by the edge portion of the support member 300. Therefore, the inflatable member 210A uniformly inflates in the lateral width wb direction of the support member 300. Accordingly, when the inflatable member 210A applies the compressive force to each of the puncture sites p1 and p2, the inflation amount in the lateral width wb direction of the support member 300 becomes uniform. Therefore, the shape of the inflatable member 210A at the time of maximum inflation and the compressive force applied to the puncture sites p1 and p2 are stabilized. Further, in the inflatable member 210A, the inflation deformation amount of the first sheet material 220 is smaller than that of the second sheet material 230. Therefore, the inflatable member 210A can be prevented from being deformed such that when the inflatable member 210A is inflated, a portion of the inflatable member 210A including the first sheet material 220 excessively protrudes toward the inner surface 300a side of the support member 300. Therefore, in the inflatable member 210A, the area of contact between the inner surface 300a of the support member 300 and the first sheet material 220 increases in a state where the inflatable member 210A is inflated. The support member 300 can firmly press the inflatable member 210 by increasing the contact area between the inner surface 300a of the support member 300 and the inflatable member 210A. Therefore, in the hemostatic device according to the modification, the compressive force applied to each of the puncture sites p1 and p2 by the inflatable member 210A is further improved.

Although the hemostatic device according to the present invention has been described above in conjunction with the embodiment and the modification, the present invention is not limited only to the content described in the specification and can be appropriately changed on the basis of the description as long as the result falls within the scope of the claims.

The shape, dimension, and the like of each portion of the hemostatic device are not particularly limited as long as hemostatic compression can be performed on the puncture site using the pressing member disposed on the puncture site, and can be appropriately changed. However, only hemostatic devices falling within the scope of claim 1 form part of the claimed invention.

The specific shapes (planar shape and cross-sectional shape) of the inflatable member and the support member are not limited to the shapes illustrated in the embodiment.

In addition, the configuration of the pressing member that applies the compressive force to the puncture site is not limited to the inflatable member (balloon). The pressing member may include, for example, a resin material such as plastic configured to be able to apply a compressive force to the puncture site, a member made of gel or the like, an elastic material such as a sponge-like substance, an assembly of fibers such as cotton, metal, a member having a predetermined three-dimensional shape (spherical, ellipsoid, triangular pyramid, or the like), or a combination thereof as appropriate.

The present application is based on Japanese Patent Application No. 2022-44656 filed on March 18, 2022, the disclosure content of which is hereby referenced.

### Reference Signs List

10 Hemostatic device
100 Cover member
110 Main body portion of cover member
110a Inner surface of main body portion
110b Outer surface of main body portion
120 Sheet material
130 Holding portion
200 Pressing member
200A Pressing member
210 Inflatable member
210A Inflatable member
210a Inner cavity of inflatable member
211 Edge portion of inflatable member
220 First sheet material
221 Edge portion of first sheet material
230 Second sheet material
231 Edge portion of second sheet material
260 Marker portion
281 Injection portion
300 Support member
300a Inner surface of support member
300b Outer surface of support member
301 Upper end portion
302 Lower end portion
302a Flat portion
303 Side surface portion
304 Side surface portion
305 Intermediate portion
410 First band body portion
420 Second band body portion
430 Third band body portion
510 First securing site
520 Second securing site
530 Third securing site
540 Fourth securing site
600 Introducer
C Center line of first band body portion
c1 Center of inflatable member
wa Longitudinal width of inflatable member
wb Lateral width of inflatable member
t1 Thickness of side surface portion
G1 Gravity center of support member
w1 Width of support member
w1' Width of upper end portion
w1" Width of lower end portion
H Hand
H1 Right hand
Ar Forearm portion
fb Inter-finger portion
Sb Snuff box
B1 Metacarpal bone of index finger
B2 Metacarpal bone of thumb
T1 Extensor pollicis longus muscle tendon
T2 Extensor pollicis brevis muscle tendon
V1 Blood vessel (distal radial artery)
V2 Artery located in snuff box
p1 First puncture site (puncture site)
p2 Second puncture site (puncture site)

## Claims

1. A hemostatic device (10) comprising:
a cover member (100) configured to cover a puncture site formed on a patient; and
a pressing member (200, 210, 210A) connected to the cover member and configured to compress the puncture site,
wherein
the cover member includes a main body portion where the pressing member is located, a first band body portion (410) configured to be disposed between fingers of the patient and extending from the main body portion in a first direction, a second band body portion (420) extending from the main body portion in a second direction different from the first direction, and a third band body portion (430) facing the second band body portion across the pressing member and extending from the main body portion in a third direction different from the first direction and the second direction,
the main body portion includes a support member (300) formed of a material harder than the pressing member and configured to overlap the pressing member,
the support member includes an upper end portion (301) located on the first band body portion side and forming an end portion of the support member, a lower end portion (302) forming an end portion opposite to the upper end portion, and a side surface portion (303,304) connecting the upper end portion and the lower end portion,
the lower end portion has a flat portion (302a) having a linear shape in plan view, and
an outer surface (300b) of the support member is curved toward the second band body portion side and the third band body portion side,
**characterized in that** the support member (300) is configured such that a width of the support member decreases from the upper end portion (301) toward the lower end portion (302), and a thickness of the side surface portion decreases from the upper end portion (301) toward the lower end portion (302).

2. The hemostatic device according to claim 1, wherein
the support member has a position serving as a gravity center (G1) of an outer shape of the support member in plan view,
the gravity center is located on the upper end portion side with respect to an intermediate portion located between the upper end portion and the lower end portion, and
the pressing member is disposed on the cover member in a state where a center of the pressing member is located on the lower end portion side with respect to the gravity center.

3. The hemostatic device according to claim 1 or 2,
wherein
the support member is bilaterally symmetrical relative to a center line (C) of the first band body portion in the main body portion, and
an inner surface (300a) of the support member is curved in a protruding shape in a direction away from the pressing member.

4. The hemostatic device according to claim 1 or 2, wherein
an inner surface (300a) of the support member is curved in a protruding shape in a direction away from the pressing member, and
a curvature radius of the inner surface of the upper end portion is smaller than a curvature radius of the inner surface of the lower end portion.

5. The hemostatic device according to any one of claims 1 to 4, wherein
the pressing member is an inflatable member inflatable by injection of a fluid,
the inflatable member has a longitudinal width (wa) passing through a center of the inflatable member in a state where the inflatable member is deflated, and
a lateral width (wb) orthogonal to the longitudinal width and passing through the center of the inflatable member,
the lateral width is shorter than the longitudinal width, and
the support member has a width of the upper end portion that is longer than the lateral width and a width of the lower end portion that is shorter than the lateral width.

6. The hemostatic device according to claim 5, wherein
the inflatable member is formed by connecting edge portions of a first sheet material and a second sheet material,
the first sheet material is connected to the cover member at a position overlapping the support member,
the edge portion of the second sheet material is located at an edge portion of the support member, and
the inflatable member is configured such that a portion including the second sheet material has a larger inflation deformation amount than a portion including the first sheet material in a state where the inflatable member is inflated.

## Patentansprüche

1. Hämostatische Vorrichtung (10), umfassend:
ein Abdeckelement (100), das so ausgebildet ist, dass es eine an einem Patienten gebildete Einstichstelle abdeckt; und
ein Druckelement (200, 210, 210A), das mit dem Abdeckelement verbunden und dazu ausgebildet ist, dass es die Einstichstelle zusammendrückt, wobei
das Abdeckelement einen Hauptkörperabschnitt, in dem sich das Druckelement befindet, einen ersten Bandkörperabschnitt (410), der so ausgebildet ist, dass er zwischen den Fingern des Patienten angeordnet wird und sich von dem Hauptkörperabschnitt in einer ersten Richtung erstreckt, einen zweiten Bandkörperabschnitt (420), der sich von dem Hauptkörperabschnitt in einer zweiten Richtung erstreckt, welche sich von der ersten Richtung unterscheidet, und einen dritten Bandkörperabschnitt (430), welcher dem zweiten Bandkörperabschnitt über das Druckelement hinweg zugewandt ist und sich von dem Hauptkörperabschnitt in einer dritten Richtung erstreckt, die sich von der ersten und der zweiten Richtung unterscheidet,
der Hauptkörperabschnitt ein Stützelement (300) umfasst, das aus einem Material gebildet ist, das härter ist als das Druckelement, und so ausgebildet ist, dass es das Druckelement überlappt,
das Stützelement einen oberen Endabschnitt (301), der auf der Seite des ersten Bandkörperabschnitts angeordnet ist und einen Endabschnitt des Stützelements bildet, einen unteren Endabschnitt (302), der einen dem oberen Endabschnitt gegenüberliegenden Endabschnitt bildet, und einen Seitenflächenabschnitt (303, 304) umfasst, welcher den oberen Endabschnitt und den unteren Endabschnitt verbindet,
wobei der untere Endabschnitt einen flachen Abschnitt (302a) aufweist, der in der Draufsicht eine lineare Form aufweist, und
wobei eine Außenfläche (300b) des Stützelements zu der Seite des zweiten Bandkörperabschnitts und zu der Seite des dritten Bandkörperabschnitts hin gekrümmt ist,
**dadurch gekennzeichnet, dass** das Stützelement (300) derart ausgebildet ist, dass sich eine Breite des Stützelements von dem oberen Endabschnitt (301) in Richtung zu dem unteren Endabschnitt (302) hin verringert, und eine Dicke des Seitenflächenabschnitts sich von dem oberen Endabschnitt (301) in Richtung zu dem unteren Endabschnitt (302) hin verringert.

2. Hämostatische Vorrichtung nach Anspruch 1, wobei
das Stützelement eine Position aufweist, die in der Draufsicht als ein Schwerpunkt (G1) einer Außenform des Stützelements dient,
der Schwerpunkt in Bezug auf einen zwischen dem oberen Endabschnitt und dem unteren Endabschnitt angeordneten Zwischenabschnitt auf der Seite des oberen Endabschnitts angeordnet ist, und
das Druckelement auf dem Abdeckelement in einem Zustand angeordnet ist, in welchem sich ein Mittelpunkt des Druckelements in Bezug auf den Schwerpunkt auf der Seite des unteren Endabschnitts befindet.

3. Hämostatische Vorrichtung nach Anspruch 1 oder 2, wobei
das Stützelement relativ zu einer Mittellinie (C) des ersten Bandkörperabschnitts in dem Hauptkörperabschnitt beidseitig symmetrisch ist und
eine Innenfläche (300a) des Stützelements in einer Richtung, die von dem Druckelement wegführt, in einer hervorstehenden Form gekrümmt ist.

4. Hämostatische Vorrichtung nach Anspruch 1 oder 2, wobei
eine Innenfläche (300a) des Stützelements in einer Richtung, die von dem Druckelement wegführt, in einer hervorstehenden Form gekrümmt ist, und
ein Krümmungsradius der Innenfläche des oberen Endabschnitts kleiner ist als ein Krümmungsradius der Innenfläche des unteren Endabschnitts.

5. Hämostatische Vorrichtung nach einem der Ansprüche 1 bis 4, wobei
das Druckelement ein aufblasbares Element ist, das durch Injektion eines Fluids aufgeblasen werden kann,
das aufblasbare Element eine longitudinale Breite (wa) aufweist, die in einem Zustand, in welchem das aufblasbare Element entleert ist, durch einen Mittelpunkt des aufblasbaren Elements verläuft, und
eine laterale Breite (wb) aufweist, die senkrecht zu der longitudinalen Breite verläuft und durch den Mittelpunkt des aufblasbaren Elements verläuft,
die laterale Breite kürzer ist als die longitudinale Breite, und
das Stützelement eine Breite des oberen Endabschnitts aufweist, die größer ist als die laterale Breite, sowie eine Breite des unteren Endabschnitts, die kleiner ist als die laterale Breite.

6. Hämostatische Vorrichtung nach Anspruch 5, wobei
das aufblasbare Element durch das Verbinden von Randabschnitten eines ersten Flächenmaterials und eines zweiten Flächenmaterials gebildet ist,
das erste Flächenmaterial an einer Position, an der es das Stützelement überlappt, mit dem Abdeckelement verbunden ist,
der Randabschnitt des zweiten Flächenmaterials an einem Randabschnitt des Stützelements angeordnet ist, und
das aufblasbare Element derart ausgebildet ist, dass ein Abschnitt, welcher das zweite Flächenmaterial umfasst, in einem Zustand, in dem das aufblasbare Element aufgeblasen ist, einen größeren Aufblas-Verformungsbetrag aufweist als ein Abschnitt, welcher das erste Flächenmaterial umfasst.

## Revendications

1. Dispositif hémostatique (10) comprenant :
un organe de recouvrement (100) configuré pour recouvrir un site de ponction formé sur un patient ; et
un organe de pression (200, 210, 210A) relié à l'organe de recouvrement et configuré pour comprimer le site de ponction,
dans lequel
l'organe de recouvrement comporte une partie de corps principal où l'organe de pression est situé, une première partie de corps de bande (410) configurée pour être disposée entre les doigts du patient et s'étendant depuis la partie de corps principal dans une première direction, une deuxième partie de corps de bande (420) s'étendant depuis la partie de corps principal dans une deuxième direction différente de la première direction, et une troisième partie de corps de bande (430)
faisant face à la deuxième partie de corps de bande à travers l'organe de pression et s'étendant depuis la partie de corps principal dans une troisième direction différente de la première direction et de la deuxième direction,
la partie de corps principal comporte un organe de support (300) formé d'un matériau plus dur que l'organe de pression et configuré pour chevaucher l'organe de pression,
l'organe de support comporte une partie d'extrémité supérieure (301) située sur le côté de la première partie de corps de bande et formant une partie d'extrémité de l'organe de support, une partie d'extrémité inférieure (302) formant une partie d'extrémité opposée à la partie d'extrémité supérieure, et une partie de surface latérale (303, 304) reliant la partie d'extrémité supérieure et la partie d'extrémité inférieure,
la partie d'extrémité inférieure présente une partie plate (302a) présentant une forme linéaire en vue de dessus, et une surface externe (300b) de l'organe de support est courbée vers le côté de la deuxième partie de corps de bande et le côté de la troisième partie de corps de bande,
**caractérisé en ce que** l'organe de support (300) est configuré de sorte qu'une largeur de l'organe de support diminue depuis la partie d'extrémité supérieure (301) vers la partie d'extrémité inférieure (302), et une épaisseur de la partie de surface latérale diminue depuis la partie d'extrémité supérieure (301) vers la partie d'extrémité inférieure (302).

2. Dispositif hémostatique selon la revendication 1, dans lequel
l'organe de support présente une position servant de centre de gravité (G1) d'une forme extérieure de l'organe de support en vue de dessus,
le centre de gravité est situé sur le côté de la partie d'extrémité supérieure par rapport à une partie intermédiaire située entre la partie d'extrémité supérieure et la partie d'extrémité inférieure, et
l'organe de pression est disposé sur l'organe de couvercle dans un état où un centre de l'organe de pression est situé sur le côté de la partie d'extrémité inférieure par rapport au centre de gravité.

3. Dispositif hémostatique selon la revendication 1 ou 2, dans lequel
l'organe de support est bilatéralement symétrique par rapport à une ligne centrale (C) de la première partie de corps de bande dans la partie de corps principal, et
une surface interne (300a) de l'organe de support est courbée en une forme saillante dans une direction éloignée de l'organe de pression.

4. Dispositif hémostatique selon la revendication 1 ou 2, dans lequel
une surface interne (300a) de l'organe de support est courbée en une forme saillante dans une direction éloignée de l'organe de pression, et
un rayon de courbure de la surface interne de la partie d'extrémité supérieure est plus petit qu'un rayon de courbure de la surface interne de la partie d'extrémité inférieure.

5. Dispositif hémostatique selon l'une quelconque des revendications 1 à 4, dans lequel
l'organe de pression est un organe gonflable pouvant être gonflé par injection d'un fluide,
l'organe gonflable présente une largeur longitudinale (wa) traversant un centre de l'organe gonflable dans un état où l'organe gonflable est dégonflé, et
une largeur latérale (wb) orthogonale à la largeur longitudinale et traversant le centre de l'organe gonflable,
la largeur latérale est plus courte que la largeur longitudinale, et
l'organe de support présente une largeur de la partie d'extrémité supérieure qui est plus longue que la largeur latérale et une largeur de la partie d'extrémité inférieure qui est plus courte que la largeur latérale.

6. Dispositif hémostatique selon la revendication 5, dans lequel
l'organe gonflable est formé par liaison des parties de bord d'un premier matériau de feuille et d'un second matériau de feuille,
le premier matériau de feuille est relié à l'organe de recouvrement à une position chevauchant l'organe de support,
la partie de bord du second matériau de feuille est située au niveau d'une partie de bord de l'organe de support, et l'organe gonflable est configuré de sorte qu'une partie comportant le second matériau de feuille présente une plus grande quantité de déformation par gonflage qu'une partie comportant le premier matériau de feuille dans un état où l'organe gonflable est
gonflé.
